(19) Europäisches Patentamt
European Patent Office
Office européen des brevets

(11) Numéro de publication: 0 385 850 A2

(12) # DEMANDE DE BREVET EUROPEEN

(21) Numéro de dépôt: 90400537.8

(22) Date de dépôt: 27.02.90

(51) Int. Cl.5: C07D 401/14, A61K 31/415, C07D 471/04, C07D 487/04, C07D 403/14, C07D 405/14, C07D 513/04, A61K 31/41

(30) Priorité: 03.03.89 FR 8902833

(43) Date de publication de la demande: 05.09.90 Bulletin 90/36

(84) Etats contractants désignés: AT BE CH DE DK ES FR GB GR IT LI LU NL SE

(71) Demandeur: LABORATOIRES UPSA
1 bis, rue du Docteur Camille Bru
F-47000 Agen(FR)

(72) Inventeur: Bru-Magniez, Nicole
24-26, avenue Raphael
F-75016 Paris(FR)
Inventeur: Güngör, Timur
47, boulevard Solférino
F-92500 Rueil-Malmaison(FR)
Inventeur: Lacrampe, Jean
14-16, Allée Saint Cucufa
F-92420 Vaucresson(FR)
Inventeur: Launay, Michèle
11, rue Diderot
F-92500 Rueil-Malmaison(FR)
Inventeur: Teulon, Jean-Marie
13, avenue Guibert
F-78170 La Celle St Cloud(FR)

(74) Mandataire: Portal, Gérard et al
Cabinet Beau de Loménie 55, rue d'Amsterdam
F-75008 Paris(FR)

(54) **Nouveaux dérivés de benzimidazoles et azabenzimidazoles, leurs procédés de préparation, intermédiaires de synthèse, compositions pharmaceutiques les contenant, utiles notamment pour le traitement des maladies cardiovasculaires, et les ulcères duodénaux.**

(57) La présente invention concerne les produits de formule :

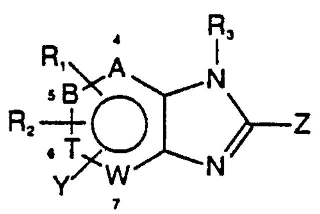

FORMULE (I)

et leur formes tautomères dans laquelle :
-Y se trouve en position 4,5,6 ou 7 du noyau benzimidazole ou azabenzimidazole et représente un dérivé de l'imidazole, du benzimidazole, du triazole ou de l'imidazothiazole pouvant être ou non substitué par des groupements tels que :
Halogène, $COR_5$, $OR_5$, $SR_5$, $COOR_5$ ($R_5$ étant un atome d'hydrogène, un radical alkyle inférieur), un radical alkyle inférieur substitué ou non par des groupements halogène, $OR_5$, $SR_5$, $COOR_5$, $NHR_5$, $NHCOR_5$,

EP 0 385 850 A2

$COR_5$, $R_5$ étant défini comme ci-dessus.
-Z peut représenter un cycle phényle ou pyridyle lié au benzimidazole ou à l'azabenzimidazole directement ou indirectement par l'intermédiaire d'un atome d'azote non substitué ou substitué par un radical alkyle inférieur en particulier l'aniline ou l'aminopyridine. Le cycle phényle ou pyridyle peut être ou non substitué notamment par un ou plusieurs radicaux alkyles inférieurs, un ou plusieurs atomes d'halogènes, un ou plusieurs groupements $OR_5$, $SR_5$, $SOR_5$, $NHCOR_5$, $NHR_5$ ($R_5$ étant défini comme ci-dessus), un hétérocycle de 5 à 10 atomes comportant 1 à 3 hétéroéléments choisis parmi l'azote, l'oxygène ou le soufre,
Z peut encore représenter un groupement OH, SH, $SR_6$ ou $SOR_6$, $R_6$ étant un alkyle inférieur, un alkényle en $C_2$-$C_8$, en particulier un allyle ou un alkynyle en $C_2$-$C_8$, en particulier un propargyle.
-$R_1$ et $R_2$ représentent simultanément ou non l'atome d'hydrogène, l'atome d'halogène ou un groupement $CF_3$, $NO_2$, $NHR_4$, $NHCOR_4$, $OR_4$,$SR_4$ ($R_4$ représentant un atome d'hydrogène ou un radical alkyle inférieur) ou un alkyle inférieur et peuvent se trouver en position 4,5,6,7 du benzimidazole ou de l'azabenzimidazole.
-$R_3$ représente l'atome d'hydrogène et peut représenter un radical alkyle inférieur ou un groupement benzyle lorsque Z représente un groupement OH, SH, $SR_6$ ou $SOR_6$.
-A, B, T, W peuvent représenter un atome de carbone ou un hétéroélément comme l'azote.
- et les médicaments en contenant.

# Nouveaux dérivés de benzimidazoles et d'azabenzimidazoles, leurs procédés de préparation, intermédiaires de synthèse, compositions pharmaceutiques les contenant, utiles notamment pour le traitement des maladies cardiovasculaires, et des ulcères duodénaux.

La présente invention concerne en tant que produits nouveaux les dérivés de benzimidazoles et d'azabenzimidazoles de formule générale (I) ci-dessous et leurs formes tautomères, et éventuellement leurs sels d'addition d'acides en particulier les sels d'addition d'acides non toxiques ou pharmacologiquement acceptables. Les composés en question présentent un profil pharmacologique très intéressant dans la mesure où ils sont doués de propriétés cardiotoniques, vasodilatatrices, anti-hypertensives et anti-agrégantes plaquettaires. Ils sont donc particulièrement indiqués dans le traitement des maladies cardiovasculaires et en particulier dans le traitement de l'insuffisance cardiaque.

De plus, certains dérivés de l'invention possèdent des propriétés anti-ulcères. Ils sont donc revendiqués également pour cette activité et leur utilisation dans le traitement des ulcères gastroduodénaux.

La présente invention concerne également le procédé de préparation des dits produits et leurs applications en thérapeutique. Elle concerne en outre les composés intermédiaires nouveaux permettant la synthèse des dits produits.

On connaît par le document US-A-3 336 192 (GB 1,085,634) des dérivés de benzazoles à activité anthelmintique, substitués en position 5 (6) et éventuellement en position 2. Les substituants en position 5 ou 6 sont en général des radicaux hétéroaromatiques, en particulier imidazolyle, thiazolyle, isothiazolyle, 1,2,5-thiadiazolyle, pyrryle, furyle et thiényle. Le substituant en position 2 est un noyau hétéroaromatique penta ou hexagonal contenant de 1 à 3 hétéroatomes choisis parmi l'oxygène le soufre et/ou l'azote. Parmi les radicaux contenant l'azote sont cités le radical pyrryle ou pyridyle, pour ceux contenant de l'oxygène, le radical furyle et pour ceux contenant le soufre, le radical thiényle. Les substituants préférés sont les groupes thiazolyle, isothiazolyle et thiadiazolyle.

Les combinaisons de substituants effectivement décrites concernent principalement des couples thiazole et isothiazole.

Les composés les plus proches de l'invention sont respectivement le 2-(4′-thiazolyl)-5 (6)-(1′-imidazolyl) benzimidazole et le 1-acétyle-2-(3′-pyridyl)-5-(4′-1,2,5-thiadiazolyl) benzimidazole.

Les composés de l'invention les plus proches diffèrent radicalement de ceux décrits dans ce document en ce qu'ils présentent obligatoirement un substituant 2-phényle ou pyridyle combiné avec, en position 5 ou 6, un imidazolyle, alors que le document précité ne précise pas cette combinaison particulière, mais au contraire suggère des combinaisons préférées thiazolyle, isothiazolyle ou thiadiazolyle.

Or, il a été découvert par l'invention, que la combinaison précise 2-phényle ou 2-pyridyle-5 (6)-imidazole, ainsi que d'autres, procurent une activité pharmaceutique inattendue, notamment pour le traitement des maladies cardiovasculaires et des ulcères duodénaux.

D'autre part, il a également pu être observé qu'une substitution du benzimidazole, par un groupe alkyle inférieur en particulier un méthyle, en position 6 ou 7, conduisait à des dérivés particulièrement actifs par voie orale ou parentérale.

Ainsi, l'invention fournit de nouveaux dérivés de benzimidazole et d'azabenzimidazoles caractérisés en ce qu'ils répondent à la formule générale (I) :

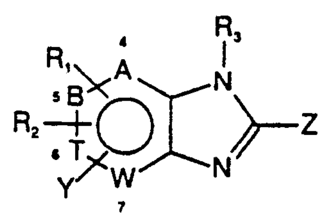

FORMULE (I)

dans laquelle :

-Y se trouve en position 4,5,6 ou 7 du noyau benzimidazole ou azabenzimidazole et représente un dérivé de l'imidazole, du benzimidazole, du triazole ou de l'imidazothiazole pouvant être ou non substitué par des groupements tels que :

halogène, $COR_5$, $OR_5$, $SR_5$, $COOR_5$ ($R_5$ étant un atome d'hydrogène, un radical alkyle inférieur), un radical alkyle inférieur substitué ou non par des groupements halogène, $OR_5$, $SR_5$,$COOR_5$, $NNR_5$, $NHCOR_5$, $COR_5$, $R_5$ étant défini comme ci-dessus.

-Z peut représenter un cycle phényle ou pyridyle lié au benzimidazole ou à l'azabenzimidazole directement ou indirectement par l'intermédiaire d'un atome d'azote non substitué ou substitué par un radical alkyle inférieur en particulier l'aniline ou l'aminopyridine. Le cycle phényle ou pyridyle peut être ou non substitué notamment par un ou plusieurs radicaux alkyles inférieurs, un ou plusieurs atomes d'halogènes, un ou plusieurs groupements $OR_5$, $SR_5$, $SOR_5$, $NHCOR_5$, $NHR_5$ ($R_5$ étant défini comme ci-dessus), un hétérocycle de 5 à 10 atomes comportant 1 à 3 hétéroéléments choisis parmi l'azote, l'oxygène ou le soufre,

Z peut encore représenter un groupement OH, SH, $SR_6$ ou $SOR_6$, $R_6$ étant un alkyle inférieur, un alkényle en $C_2$-$C_8$, en particulier un allyle ou un alkynyle en $C_2$-$C_8$, en particulier un propargyle.

-$R_1$ et $R_2$ représentent simultanément ou non l'atome d'hydrogène, l'atome d'halogène ou un groupement $CF_3$, $NO_2$, $NHR_4$, $NHCOR_4$, $OR_4$, $SR_4$ ($R_4$ représentant un atome d'hydrogène ou un radical alkyle inférieur) ou un alkyle inférieur et peuvent se trouver en position 4,5,6,7 du benzimidazole ou de l'azabenzimidazole.

-$R_3$ représente l'atome d'hydrogène et peut représenter un radical alkyle inférieur ou un groupement benzyle lorsque 2 représente un groupement OH, SH, $SR_6$ ou $SOR_6$.

-A, B, T, W peuvent représenter un atome de carbone ou un hétéro élément comme l'azote.

Dans la description et les revendications on entend par radical alkyle inférieur une chaîne de 1 à 6 atomes de carbone linéaire ou ramifiée ou cyclique en $C_3$-$C_6$.

Dans la description et les revendications, on entend par halogène un atome de chlore, de brome, d'iode ou de fluor, de préférence un atome de chlore.

Le nombre total d'atomes de carbone du groupement Z comprenant le cycle phényle ou pyridyle peut être compris entre 5 et 20.

Selon une variante de réalisation particulière, Y est l'imidazole.

Selon une autre variante, Z est la pyridine en particulier le radical pyridyl-4.

Selon encore une autre variante, Z est l'amino pyridine et en particulier l'amino-4 pyridine.

Selon encore une autre variante de réalisation de l'invention, Z est le groupement OH.

Selon encore une autre variante de réalisation, Z est le groupement SH.

Selon un mode de réalisation particulier, $R_1$ est l'atome d'hydrogène.

Selon un autre mode de réalisation particulier, $R_1$ est un radical alkyle inférieur en particulier un méthyle, et de préférence en position 6 ou 7.

Selon encore une autre variante de réalisation, $R_1$ est l'atome de chlore.

Selon une variante de réalisation, $R_1$ est le groupement trifluorométhyle

Selon un mode de réalisation particulier, $R_2$ est l'atome d'hydrogène.

Selon un autre mode de réalisation particulier, $R_2$ est un radical alkyle inférieur et en particulier un méthyle de préférence en position 7 ou 6.

Selon encore une autre variante de réalisation, $R_3$ est le radical isopropyle lorsque Z est le groupement OH ou SH.

Selon une variante, W est l'atome d'azote.

Selon une autre variante, B et W sont des atomes d'azote.

Selon une autre variante de réalisation, $R_1$ est un radical alkyle inférieur, en particulier le méthyle, en position 7, $R_2$ l'atome d'hydrogène, Y est un radical imidazolyle et Z est un radical pyridyl-4.

Des composés de l'invention particulièrement préférés sont ceux qui sont choisis parmi les produits de formule :

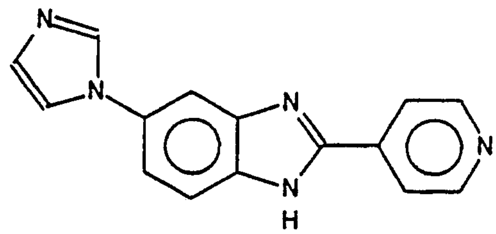

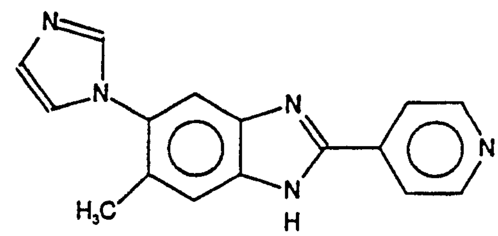

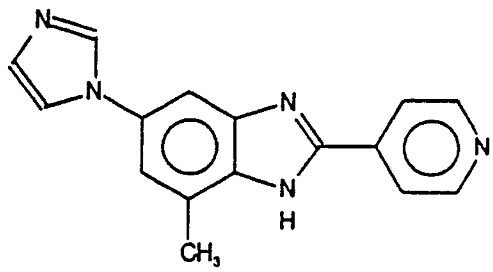

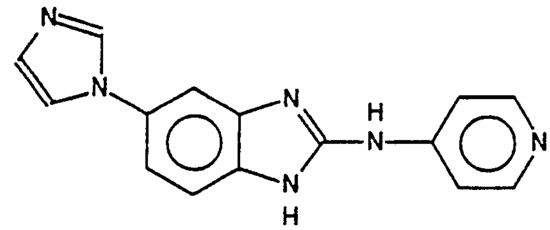

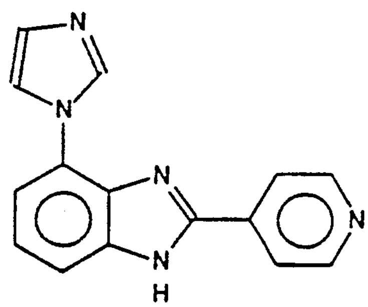

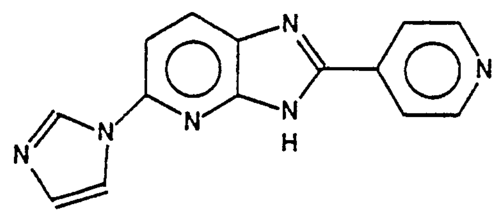

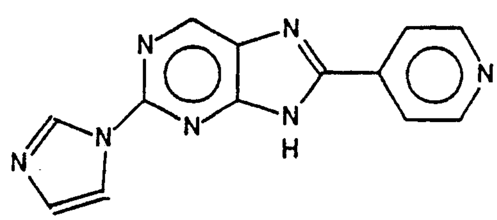

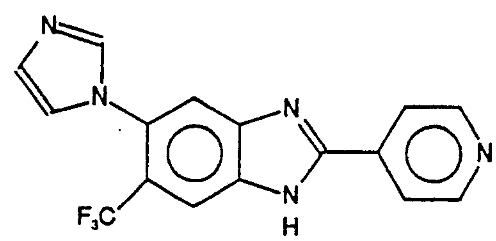

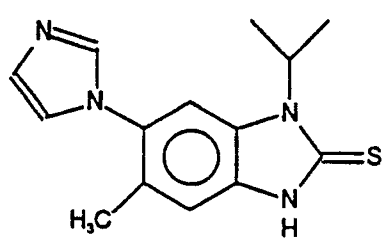

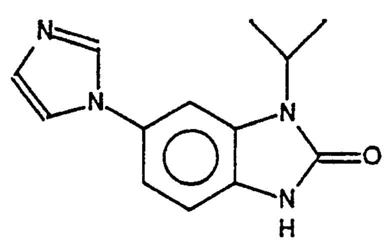

.HCL

Selon l'invention les dérivés de formule (I) où Y représente un dérivé de l'imidazole, du triazole ou du benzimidazole peuvent être préparés selon les schémas suivants :

A partir des dérivés de formule (II)

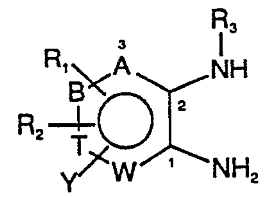

FORMULE (II)

dans laquelle A,B,T,W,$R_1$,$R_2$,$R_3$,Y sont définis comme ci-dessus

- par action de l'urée, de la thiourée ou du sulfure de carbone pour les dérivés où Z représente le groupement OH ou SH, dans un solvant organique ou sans solvant à une température comprise entre 40 et 200° C.

Pour obtenir les composés où Z représente le groupement S-$R_6$, $R_6$ étant défini comme ci-dessus, on fera réagir le dérivé où Z représente le groupement SH avec un composé halogéné de formule X-$R_6$, X étant un halogène et $R_6$ défini comme ci-dessus dans un solvant organique tel qu'un alcool ou le diméthylformamide en milieu basique à une température comprise entre 20 et 150° C.

Les dérivés où 2 représente -$SOR_6$, $R_6$ étant défini comme ci-dessus, seront obtenus par oxydation à l'aide d'un péracide; par exemple l'acide métachloroperbenzoïque, des dérivés où Z est $SR_6$ dans un solvant organique tel que le chloroforme ou le dichlorométhane à des températures comprises entre -5 et 25° C.

-dans le cas où Z représente un cycle phényle ou pyridyle, on pourra :

. soit chauffer les composés de formule (II) dans lesquels $R_3$ est l'atome d'hydrogène dans un solvant organique tel qu'un alcool avec un aldéhyde de formule Z-CHO, Z étant un cycle phényle ou pyridyle et le dérivé obtenu subira une oxydation par traitement thermique entre 150 et 200° C dans le nitrobenzène.

. soit former le benzimidazole par les méthodes classiques avec les chlorures d'acides Z-COCl, les acides Z-COOH ou les nitriles Z-CN dans lesquels Z est un cycle phényle ou pyridyle selon la référence : J.B. WRIGHT Chem. Rev. 1951, 48, 397

-dans le cas où Z représente une aniline ou une amino pyridine, les dérivés de formule (II) dans lesquels $R_3$ est l'atome d'hydrogène seront mis en réaction avec des composés de formules :

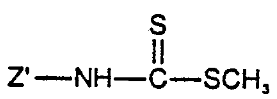 ; 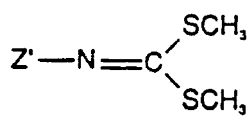 ; $Z'-N=C=S$

dans lesquels Z′ est un cycle phényle ou pyridyle, selon la méthode décrite dans les références : A. MOHSEN et coll. Synthesis 1977, 864 F. MERCHAN et coll. Synthesis 1982, 482.

Pour avoir sur le noyau phényle ou pyridyle un groupement -$SOCH_3$ on opèrera comme précédemment l'oxydation du dérivé substitué par un groupement -$SCH_3$ à l'aide d'un peracide, par exemple l'acide métachloroperbenzoïque dans un solvant tel que le chloroforme ou le dichlorométhane à une température comprise entre -50° C et 50° C.

Les composés de formule (II) pourront être obtenus par hydrogénation en présence de catalyseur tel que le nickel de Raney ou par une réduction connue Fe/HCl de nitro amines de formule(III) :

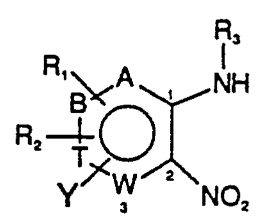

FORMULE (III)

dans laquelle A,B,T,W,$R_1$,$R_2$,$R_3$,Y sont définis comme ci-dessus. Les composés de formule (III) peuvent être obtenus par simple désacétylation de composés de formule (IV)

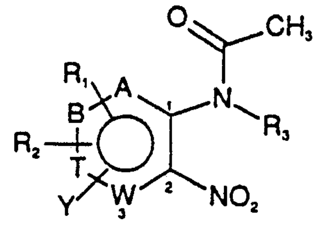

FORMULE (IV)

Les composés de formule (III) et (IV) pour lesquels Y représente un dérivé de l'imidazole, du triazole ou du benzimidazole peuvent être obtenus par action d'un dérivé de l'imidazole (ou de son sel de sodium), du triazole (ou de son sel de sodium), du benzimidazole (ou de son sel de sodium) ou du sel de sodium de l'imidazolone, dans un solvant organique tel que le diméthylformamide ou directement sans solvant par chauffage à une température comprise entre 80 et 200 °C, sur des dérivés de formule (V) ou (VI).

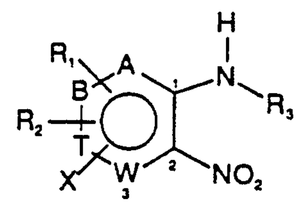

FORMULE (V)

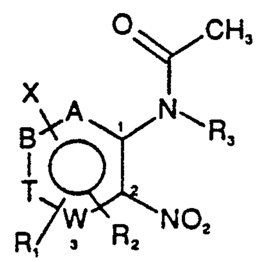

FORMULE (VI)

dans lesquelles A,B,T,W,$R_1$,$R_2$,$R_3$, sont définis comme ci-dessus et X représente l'atome d'halogène.

Les composés de formule (V) pourront être obtenus :

- soit par désacétylation de composés de formule (VI)
- soit par action d'amines de formule $R_3NH_2$, $R_3$ étant défini comme ci-dessus, sur des dérivés de formule (VII) sans solvant ou dans un solvant organique tel que l'eau ou l'alcool, à pression atmosphérique ou sous pression, à une température comprise entre 25 et 200 °C.

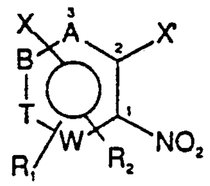

FORMULE (VII)

dans laquelle A,B,T,W,$R_1$,$R_2$ sont définis comme ci-dessus, X et X′ représentent un atome d'halogène.

Dans le cas où $R_3$ est un hydrogène, les composés de formule (V) peuvent également être obtenus soit par dégradation d'Hofmann des amides [E.S. Wallis ; J.F. Lane ; Organic Reaction 3,267 (1949)] soit par réduction partielle des composés dinitro correspondants (B.M. Wepster et P.E. Verkade Recueil des travaux chimiques des Pays Bas 1949 Vol. 68 P.105).

Les composés de formule (VI) pourront être obtenus par nitration selon des méthodes connues en soi de dérivés de l'acétamide correspondant qui sont commerciaux ou qui peuvent eux mêmes être préparés à partir de dérivés de composés aminés commerciaux par action de l'anhydride acétique ou du chlorure d'acétyle selon des méthodes connues en soi.

Les composés de formule (VII) pourront être obtenus :

-par simple nitration selon les méthodes connues en soi de dérivés commerciaux,

-à partir des amines de formule (VIII) où A,B,T,W,$R_1$,$R_2$ sont définis comme ci-dessus, X et X′ représentant un atome d'halogène

soit par oxydation avec un agent oxydant comme l'eau oxygénée, soit par l'intermédiaire de fluoroborates de diazonium grâce à des méthodes décrites en soi (Vogel A.I. Third Edition P. 595)

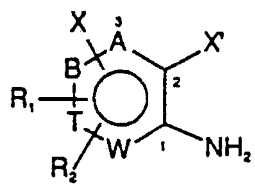

FORMULE (VIII)

Les composés (VIII) peuvent être obtenus par halogénation des amines aromatiques selon des méthodes connues en soi à partir des composés commerciaux ou décrits dans la littérature.

Les composés de formule (I) pour lesquels Y représente des dérivés de l'imidazothiazole peuvent être obtenus :

**a)** par les voies décrites précédemment, à partir de dérivés de formule (II) où A,T,$R_1$,$R_2$,$R_3$ sont définis comme ci-dessus et où Y représente un dérivé de l'imidazothiazole.

Dans ce cas les composés de formule (II) peuvent être obtenus à partir de dérivés de formule (IX) où Y est un dérivé de l'imidazothiazole, $R_1$ et $R_2$ représentent un atome d'hydrogène ou un radical alkyle inférieur, X est un halogène et A,T sont des atomes de carbone.

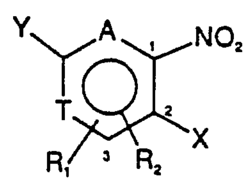

FORMULE (IX)

Ces composés de formule (IX) peuvent être obtenus par cyclisation intramoléculaire de produits de formule (X) dans laquelle $R_1$ $R_2$, $R_8$ sont des atomes d'hydrogène ou des radicaux alkyles inférieurs, X est un atome d'halogène et $R_7$ est un dérivé de l'imidazole, dans des solvants organiques tels que toluène,

xylène, DMF ou N-méthyl pyrrolidone par exemple, en présence ou non d'acide paratoluène sulfonique, ou dans du PPA, à des températures allant de 25 à 180 ° C.

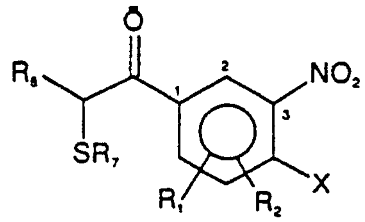

FORMULE (X)

Les composés de formule (X) sont obtenus à partir d'halogénoalkyl (halogéno nitro phényl) cétones substituées ou non sur le phényl et préparées selon des méthodes décrites dans la littérature, (Beil. 7,285) par réaction avec des composés mercaptoimidazoles selon des méthodes connues (Beil. 23,353).

**b)** par cyclisation intramoléculaire dans des conditions décrites précédemment (dans le PPA par exemple) de composés de formule (XI), dans laquelle $R_1$, $R_2$, $R_3$, $R_7$, $R_8$ sont définis comme ci-dessus,

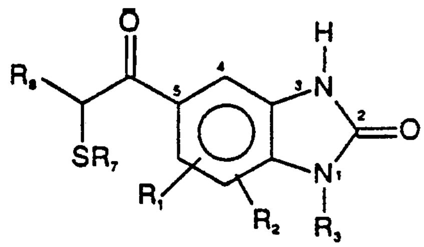

FORMULE (XI)

Ces composés de formule (XI) sont préparés par action de dérivés de mercapto-2 imidazoles sur des composés de formule (XII) selon des méthodes connues citées précédemment :

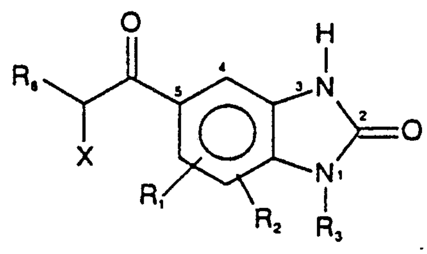

FORMULE (XII)

dans laquelle $R_1$, $R_2$, $R_3$, $R_8$ sont définis comme ci-dessus et X est un atome d'halogène.

Les composés de formule (XII) sont obtenus par réaction classique de Friedel et Crafts entre des benzimidazoles diversement substitués ou non, et des chlorures d'acides halogénés

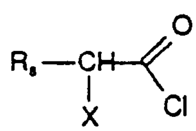

$R_8$ et X étant définis comme ci-dessus en présence d'acides de Lewis, dans des solvants comme le sulfure de carbone ou le dichlorométhane.

Des sels d'addition d'acides de certains composés de formule (I) peuvent s'obtenir par réaction de ces composés avec un acide minéral ou organique suivant une méthode connue en soi. On utilisera avantageusement les sels d'addition d'acides pharmacologiquement acceptables non toxiques.

Parmi les acides utilisables à cet effet, on citera les acides chlorhydrique, bromhydrique, sulfurique, phosphorique, 4-toluène sulfonique, méthane sulfonique, cyclohexyl sulfamique, oxalique, succinique, formique, fumarique, maléique, citrique, aspartique, cinnamique, lactique, glutamique, N-acétylaspartique, N-acétyl glutamique, ascorbique, malique, benzoïque, nicotinique et acétique.

Les nouveaux composés selon l'invention possèdent des propriétés pharmacologiques remarquables et peuvent être utilisés en thérapeutique pour le traitement des maladies cardiovasculaires et en particulier dans le traitement de l'insuffisance cardiaque puisqu'ils possèdent des propriétés cardiotoniques, vasodilatatrices, anti-hypertensives et anti-agrégantes plaquettaires.

De plus, comme il a été signalé précédemment les composés de l'invention peuvent présenter des propriétés anti-ulcères et anti-sécrétoires et pourront donc être utiles dans le traitement des ulcères gastroduénaux.

Ainsi, l'invention couvre également une composition pharmaceutique, caractérisée en ce qu'elle comprend une quantité pharmaceutiquement efficace d'au moins un composé de formule (I), tel que précédemment défini, ainsi que ses sels d'addition d'acides pharmacologiquement acceptables, éventuellement incorporé dans un excipient, véhicule ou support pharmaceutiquement acceptable.

Selon une variante de réalisation, cette composition pharmaceutique présente une activité cardiovasculaire, cardiotonique, vasodilatatrice, anti-hypertensive et anti-agrégante plaquettaire.

Selon une autre variante de réalisation, cette composition pharmaceutique présente une activité anti-secrétoire et anti-ulcère.

En thérapeutique humaine et animale, les composés de formule (I) et leurs sels d'addition d'acides non toxiques peuvent être administrés seuls ou en association avec un excipient physiologiquement acceptable sous une forme quelconque, en particulier sous forme de gélules et de comprimés par voie orale ou sous forme de soluté injectable par voie parentérale.

Comme il ressortira clairement des essais de pharmacologie donnés en fin de description, les composés selon l'invention peuvent être administrés en thérapeutique humaine dans les indications précitées par voie orale sous forme de comprimés ou de gélules dosée de 150 à 500 mg ou par voie parentérale sous forme de préparations injectables dosées de 30 à 200 mg en une ou deux prises unitaires journalières pour un adulte de poids moyen 60 à 70 kg.

En thérapeutique animale la dose journalière utilisable devrait habituellement se situer entre 3 et 30 mg par kg.

L'invention concerne encore un procédé de préparation d'une composition pharmaceutique caractérisée en ce qu'on incorpore une quantité thérapeutiquement efficace d'au moins un composé de formule (I), tel que précédemment défini, ainsi qu'éventuellement ses sels d'addition d'acides pharmacologiquement acceptables, dans un excipient, véhicule ou support pharmaceutiquement acceptable.

Selon une variante de réalisation, ce procédé de préparation concerne la préparation d'une composition pharmaceutique à activité cardiovasculaire, cardiotonique, vasodilatatrice, anti-hypertensive et anti-agrégante plaquettaire ou anti-sécrétoire ou anti-ulcère.

Selon une autre variante de réalisation de ce procédé, on prépare la composition pharmaceutique sous forme de gélules ou de comprimés administrables par voie orale ou sous forme de solutés injectables par voie parentérale.

Ces comprimés ou gélules peuvent être avantageusement dosés de 150 à 500 mg et les préparations injectables peuvent être dosées de 30 à 200 mg.

Selon encore un autre aspect, l'invention fournit un procédé de traitement thérapeutique, caractérisé en ce qu'on administre à un mammifère comprenant un animal ou un être humain une quantité thérapeutiquement efficace d'au moins un composé de formule (I), tel que précédemment défini ou éventuellement un de ses sels d'addition d'acides pharmacologiquement acceptables, éventuellement incorporé dans un excipient, véhicule ou support pharmaceutiquement acceptable.

Selon une variante de réalisation de ce procédé de traitement , on administre, en thérapeutique humaine, une dose journalière comprise entre 150 et 1000 mg sous forme de comprimés ou de gélules, ou une dose journalière de 30 à 400 mg par voie parentérale pour un être humain pesant entre 60 à 70 kg.

Selon une autre variante, en thérapeutique animale, on administre une dose journalière comprise entre 3 et 30 mg par kg.

Selon encore une variante de réalisation de l'invention, on réalise le traitement d'une maladie cardiovasculaire ou d'insuffisance cardiaque ou de l'hypertension ou encore un traitement anti-agrégant plaquettaire ou encore un traitement anti-sécrétoire ou encore un traitement anti-ulcère, notamment anti-ulcère duodénal..

D'autres caractéristiques et avantages de l'invention seront mieux compris à la lecture qui va suivre de

quelques exemples de préparation nullement limitatifs, mais donnés à titre d'illustration.

Exemple 1 **Nitro-2 chloro-3 aniline**

**Formule (V)** A = B = T = W = C, $R_1$ = $R_2$ = $R_3$ = H, X = 3-Cl

Dans une solution de 350 $cm^3$ d'eau et 33,6g de KOH on ajoute 11,5 $cm^3$ de brome. Puis en une fois on introduit 40,1g de nitro-2 chloro-3 benzamide obtenu par condensation d'ammoniaque sur le chlorure d'acide correspondant.

Laisser le mélange sous agitation pendant 45 mn. Filtrer l'insoluble pour avoir une solution limpide.

Ajouter le filtrat en une fois dans une solution de 44,8g de KOH dans 100 $cm^3$ d'eau. Chauffer l'ensemble à 70-75° C pendant 45 mn puis revenir à la température ambiante. Filtrer le précipité marron, laver à l'eau puis au pentane. Sécher et purifier par chromatographie sur gel de silice pour obtenir 10,5g de nitro-2 chloro-3 aniline.

Point de fusion : 110° C.

Exemple 2 **Dichloro-2,4 éthyl-6 aniline**

**Formule (VIII)** A = B = T = W = C, $R_1$ = H, $R_2$ = 6-Et, X = 4-Cl, X′ = 2-Cl

Une solution de 483 ml d'éthanol à 95%, 483 ml d'acide chlorhydrique concentré et 81g de dichloro-2,4 éthyl-6 acétanilide (point de fusion : 146-148° C) obtenu à partir de éthyl-2 aniline selon la méthode décrite dans J. Am. Chem. Soc. (1950) p. 2454-7, est portée au reflux pendant 14h. Concentrer puis neutraliser avec une solution de soude. Extraire à l'acétate d'éthyle, laver les phases organiques à l'eau. Après évaporation du solvant l'huile marron obtenue est purifiée par distillation pour donner 60g de dichloro-2,4 éthyl-6 aniline.

Point d'ébullition : 135-145° C sous 20 mm de mercure.

Exemple 3 **Dichloro-3,5 nitro-2 toluène**

**Formule (VII)** A = B = T = W = C, $R_1$ = H, $R_2$ = 6-$CH_3$, X = 4-Cl, X′ = 2-Cl

Placer 21,6g de dichloro-2,4 méthyl-6 aniline [J. Am. Chem. Soc. (1950) p. 2454-7] dans 32 ml de HCl concentré et 32 ml d'eau. Agiter à 50-60° C pendant 30 mn puis refroidir à 5° C et introduire goutte à goutte une solution de 9g de Na $NO_2$ dans 20 $cm^3$ d'eau de sorte que la température soit comprise entre 5 et 7° C. Puis agiter 1h à 5° C avant d'introduire goutte à goutte et à 5° C une solution de 20g de Na $BF_4$ dans 40 $cm^3$ d'eau. Filtrer le précipité blanc formé, laver à l'eau froide puis à l'isopropanol pour obtenir 28,5g de fluoroborate de diazonium correspondant.

Point de fusion ~ 200° C.

Le sel ainsi préparé est ensuite ajouté lentement dans 300 $cm^3$ d'eau contenant 100g de Na $NO_2$ et 20g de poudre de cuivre préparé fraîchement à partir de Cu $SO_4$/Zn par la méthode classique décrite dans la littérature. Filtrer le cuivre lorsque le dégagement d'azote s'arrête. Extraire le filtrat à l'éther, sécher, concentrer sous vide pour obtenir une huile rouge purifiée par distillation sous azote pour donner 14g de dichloro-3,5 nitro-2 toluène.

Température d'ébullition : 142-150° C sous 24 mm de Hg.

Point de fusion : 68° C

Exemple 4 **Dichloro-2,4 éthyl-6 nitro benzène**

**Formule (VII)** A = B = T = W = C, $R_1$ = H, $R_2$ = 6-éthyl, X = 4-Cl, X′ = 2-Cl

112g de dichloro-2,4 éthyl-6 aniline préparée selon l'exemple 2 sont traités comme à l'exemple 3 pour donner 35g de dichloro 2,4 éthyl-6 nitro benzène.
Point d'ébullition 135-140 °C sous 20 mm de mercure.

Exemple 5 **Dichloro-2,4 diméthyl-5,6 nitro benzène**

**Formule (VII)** A = B = T = W = C, $R_1$ = 5-$CH_3$, $R_2$ = 6-$CH_3$, X = 4-Cl, X′ = 2-Cl

113,2g de dichloro-2,4 diméthyl-5,6 aniline préparée selon J. Chem. Soc. (1934) p 283-7, sont traités comme à l'exemple 3 pour donner après distillation 46g de dichloro-2,4 diméthyl-5,6 nitro benzène.
Point d'ébullition 138-152 °C sous 6 mm de mercure.
Point de fusion : 72 °C.

Exemple 6 **Trichloro-2,4,5 méthyl-6 nitro benzène**

**Formule (VII)** A = B = T = W = C, $R_1$ = 5-Cl, $R_2$ = 6-$CH_3$, X = 4-Cl, X′ = 2-Cl

149,8g de trichloro-2,4,5 méthyl-6 aniline préparée selon J. Org. Chem. (1951) p. 328-33 sont traités selon l'exemple 3 pour donner après distillation sous azote 81,2g de trichloro-2,4,5 méthyl-6 nitro benzène.
Point d'ébullition : 124-132 °C sous 6 mm de mercure
Point de fusion : 66 °C.

Exemple 7 **Dichloro-2,4 méthyl-5 nitro benzène**

**Formule (VII)** A = B = T = W = C, $R_1$ = 5-$CH_3$, X = 4-Cl, X′ = 2-Cl

Dissoudre 8,2 g de nitrite de sodium dans 90 ml d'acide sulfurique. Introduire dans le mélange précédent une solution de 20 g de chloro-5 méthyl-4 nitro-2 aniline dans 220 ml d'acide acétique sans que la température ne dépasse 40 °C. Chauffer 30 mn supplémentaires à 40 °C. Ce mélange est alors additionné lentement à une solution froide (5 °C) de 23,6 g de chlorure cuivreux dans 220 ml d'acide chlorhydrique. Chauffer au bain marie jusqu'à ce qu'il n'y ait plus de dégagement gazeux. Ajouter alors 600 ml d'eau et laisser le mélange à froid pendant une nuit. Le solide formé est filtré, lavé à l'eau puis repris à l'éther. La phase organique ainsi obtenue est lavée à l'eau basique, séchée, concentrée pour obtenir 17,9 g de dichloro-2,4 méthyl-5 nitro benzène sous forme d'huile qui cristallise.
Point de fusion : 50 °C.

Exemple 8 **N-isopropyl chloro-5 méthyl-4 nitro-2 aniline**

**Formule (V)** A = B = T = W = C, $R_1$ = 4-$CH_3$, $R_2$ = H, $R_3$ = iPr, X = 5-Cl

Dans un autoclave on place 17,9 g de dichloro-2,4 méthyl-5 nitro benzène, 100 ml d'éthanol, 40 ml d'isopropylamine. L'ensemble est chauffé à 100 °C pendant 24 h et sous pression. Evaporer l'éthanol. Après purification sur gel de silice on obtient 11 g de N-isopropyl chloro-5 méthyl-4 nitro-2 aniline sous forme d'huile orangée utilisée brute dans l'opération ultérieure.

Exemple 9 **N-méthyl chloro-5 nitro-2 aniline**

**Formule (V)** A = B = T = W = C, $R_1$ = $R_2$ = H, $R_3$ = $CH_3$, X = 5-Cl

A une solution refroidie de 115,2 g de dichloro-2,4 nitro benzène dans 400ml d'éthanol, on ajoute 300 ml d'une solution 8 M de monométhylamine dans l'éthanol. L'ensemble est chauffé à 50 °C pendant 10 h. Refroidir, essorer le solide obtenu. Laver à l'eau puis à l'isopropanol, sécher pour obtenir 106,7 g de N-méthyl chloro-5 nitro-2 aniline.
Point de fusion : 106-107 °C

Exemple 10 **N-isopropyl fluoro-5 nitro-2 aniline**

**Formule (V)** A = B = T = W = C, $R_1$ = $R_2$ = H, $R_3$ = iPr, X = 5-F

25g de difluoro-2,4 nitrobenzène sont dissous dans 100 ml d'éthanol. On ajoute alors 67 ml d'isopropylamine lentement car la réaction est très exothermique, puis la solution est chauffée 2 h au reflux. Le milieu est concentré, le résidu repris à l'eau, extrait au chloroforme. La phase organique est séchée, concentrée et le résidu chromatographié sur gel de silice pour donner 15 g de N-isopropyl fluoro-5 nitro-2 aniline.
Point de fusion : 72 °C.

Exemple 11 **N-benzyl fluoro-5 nitro-2 aniline**

**Formule (V)** A = B = T = W = C, $R_1$ = $R_2$ = H, $R_3$ = $CH_2$ Ø, X = 5-F

Selon le mode opératoire décrit dans l'exemple 10 en faisant réagir la benzylamine et le difluoro-2,4 nitro benzène on obtient la N-benzyl fluoro-5 nitro-2 aniline.
Point de fusion : 100 °C

Exemple 12 **Chloro-5 méthyl-4 nitro-2 acetanilide**

**Formule (VI)** A = B = T = W = C, $R_1$ = 4-$CH_3$, $R_2$ = $R_3$ = H, X = 5-Cl

A une solution de 357 g de chloro-3 méthyl-4 acétanilide dans 700 ml d'anhydride acétique et 350 ml d'acide acétique, refroidie à -5 °C, on introduit goutte à goutte un mélange de 128 ml d'acide nitrique et 175 ml d'acide acétique. La température est maintenue entre -5 ° et 0 °C. Agiter 2 h supplémentaires à 0 °C puis verser la solution dans l'eau. Triturer le précipité, essorer, laver à l'eau puis à l'isopropanol. Recristalliser dans l'éthanol pour obtenir 254,5 g de chloro-5 méthyl-4 nitro-2 acétanilide.
Point de fusion : 114 °C

Exemple 13 **N-méthyl chloro-5 méthyl-4 nitro-2 acétanilide**

**Formule (VI)** A = B = T = W = C, $R_1$ = 4-$CH_3$, $R_2$ = H, $R_3$ = $CH_3$, X = 5-Cl

Dans un tricol muni d'une agitation, d'un réfrigérant et d'une ampoule à addition, on place 37,2 g de chloro-5 méthyl-4 nitro-2 acétanilide préparé selon l'exemple 12 en solution dans 200 ml de méthyl éthyl cétone. On ajoute alors 27 g de $K_2CO_3$ et 30,6 ml d'iodure de méthyle. La suspension est chauffée au reflux 24 h puis après addition de 30,6 ml d'iodure de méthyle supplémentaires de nouveau 20 h. Après refroidissement l'insoluble est filtré et le filtrat concentré. Le résidu est chromatographié sur gel de silice pour donner 10g de produit de départ et 27,7 g de N-méthyl chloro-5 méthyl-4 nitro-2 acétanilide sous forme d'huile jaune utilisée brute dans l'opération ultérieure.

Exemple 14 **Chloro-5 méthyl-4 nitro-2 aniline**

**Formule (V)** A = B = T = W = C, $R_1$ = 4-$CH_3$, $R_2$ = $R_3$ = H, X = 5-Cl

Dissoudre 26 g de sodium dans 970 ml de méthanol. Introduire 254,5 g de chloro-5 méthyl-4 nitro-2 acétanilide préparé selon l'exemple 12 et agiter 3 h à température ambiante. Verser dans de l'eau. Triturer, essorer le solide obtenu. Laver à l'eau puis à l'isopropanol pour obtenir 200,5 g de chloro-5 méthyl-4 nitro-2 aniline.
Point de fusion : 166° C

Exemple 15 **Chloro-5 méthyl-6 nitro-2 aniline**

**Formule (V)** A = B = T = W = C, $R_1$ = 6-$CH_3$, $R_2$ = $R_3$ = H, X = 5-Cl

On porte au reflux pendant 23h, un mélange contenant 400 $cm^3$ d'acide chlorhydrique concentré, 400 $cm^3$ d'éthanol à 95% et 72,4g de chloro-5 méthyl-6 nitro-2 acétanilide, préparé selon Beilstein 12 II 461. Après refroidissement filtrer le précipité. Concentrer le filtrat pour obtenir un deuxième lot. Réunir les précipités obtenus. Laver à l'eau alcalinisée pour obtenir 57,6g de chloro-5 méthyl-6 nitro-2 aniline.
Point de fusion : 153° C.

Exemple 16 **Chloro-5 méthyl-3 nitro-2 aniline**

**Formule (V)** A = B = T = W = C, $R_1$ = 3-$CH_3$, $R_2$ = $R_3$ = H, X = 5-Cl

Dans un autoclave on place 116,7g de dichloro-3,5 nitro-2 toluène préparé à l'exemple 3 et 500 $cm^3$ de méthoxy-2 éthanol. Refroidir le mélange à -35° C et ajouter 145 $cm^3$ d'ammoniac liquide.

L'autoclave est porté à 150° C pendant 48h. Refroidir. Le composé brut obtenu est purifié par chromatographie sur gel de silice pour donner 57,6g de chloro-5 méthyl-3 nitro-2 aniline
Point de fusion : 76° C.

Selon l'exemple 16, les composés suivants des exemples 17 à 19 ont été préparés :

Exemple 17 **Chloro-5 éthyl-3 nitro-2 aniline**

**Formule (V)** A = B = T = W = C, $R_1$ = 3-$C_2H_5$, $R_2$ = $R_3$ = H, X = 5-Cl

Huile utilisée sans purification supplémentaire au stade ultérieur.

Exemple 18 **Dichloro-4,5 méthyl-3 nitro-2 aniline**

**Formule (V)** A = B = T = W = C, $R_1$ = 4-Cl, $R_2$ = 3-$CH_3$, $R_3$ = H, X = 5-Cl

Point de fusion : 122° C.

Exemple 19 **Chloro-5 nitro-2 trifluorométhyl-4 aniline**

**Formule (V)** A = B = T = W = C, $R_1$ = 4-$CF_3$, $R_2$ = $R_3$ = H, X = 5-Cl

Point de fusion : 114° C.

Exemple 20 **Chloro-5 diméthyl-3,4 nitro-2 aniline**

De la même manière qu'à l'exemple 16 mais en portant l'autoclave à 200 °C pendant 48h, on obtient le chloro-5 diméthyl-3,4 nitro-2 aniline.
Point de fusion : 90 °C.

Exemple 21 : **(Imidazolyl-1)-5 méthyl-4 nitro-2 acétanilide**

**Formule (IV)** A = B = T = W = C, $R_1$ = 4-$CH_3$, $R_2$ = $R_3$ = H, Y = (imidazolyl-1)-5

20,6 g de chloro-5 méthyl-4 nitro-2 acétanilide préparé à l'exemple 12 et 9,2 g d'imidazole sont mélangés intimement et chauffés 2 h à 180 °C. Après avoir refroidi, le milieu est versé dans un mélange de 100 ml de chloroforme et 100 ml d'eau sous agitation. La phase organique est décantée et extraite avec une solution diluée d'acide chlorhydrique. La phase acide est basifiée avec de la soude 1N et extraite avec du chloroforme. Le chloroforme est séché sur sulfate de magnésium, puis concentré.

On obtient ainsi 10,8 g de (imidazolyl-1)-5 méthyl-4 nitro-2 acétanilide.
Point de fusion F = 146 °C.

Exemple 22 **N-méthyl (imidazolyl-1)-5 méthyl-4 nitro-2 acétanilide**

**Formule (IV)** A = B = T = W = C, $R_1$ = 4-$CH_3$, $R_1$ = H, $R_3$ = $CH_3$, Y = (imidazolyl-1)-5

14,2 g de N-méthyl chloro-5 méthyl-4 nitro-2 acétanilide synthétisés à l'exemple 13 sont traités selon le mode opératoire de l'exemple 21 par 8 g d'imidazole. On obtient ainsi 14,7 g de N-méthyl (imidazolyl-1)-5 méthyl-4 nitro-2 acétanilide sous forme d'huile utilisée brute dans l'opération ultérieure.

Exemple 23 **N-méthyl (méthyl-2 imidazolyl-1)-5 méthyl-4 nitro-2 acétanilide**

**Formule (IV)** A = B = T = W = C, $R_1$ = 4-$CH_3$, $R_2$ = H, $R_3$ = $CH_3$, Y = (méthyl-2 imidazolyl-1)-5

De la même manière que pour l'exemple 21 mais en remplaçant l'imidazole par du méthyl-2 imidazole on obtient le N-méthyl (méthyl-2 imidazolyl-1)-5 méthyl-4 nitro-2 acétanilide sous forme d'huile utilisée brute dans l'opération ultérieure.

Exemple 24 **N-méthyl (imidazolyl-1)-5 méthyl-4 nitro-2 aniline**

**Formule (III)** A = B = T = W = C, $R_1$ = 4-$CH_3$, $R_2$ = H, $R_3$ = $CH_3$, Y = (imidazolyl-1)-5

Le N-méthyl (imidazolyl-1)-5 méthyl-4 nitro-2 acétanilide synthétisé à l'exemple 22 est traité selon l'exemple 14 pour donner la N-méthyl (imidazolyl-1)-5 méthyl-4 nitro-2 aniline.
Point de fusion : 219 °C.

Exemple 25 **(Imidazolyl-1)-5 méthyl-4 nitro-2 aniline**

**Formule (III)** A = B = T = W = C, $R_1$ = 4-$CH_3$, $R_2$ = H, $R_3$ = H, Y = (imidazolyl-1)-5

On chauffe à 180 °C un mélange contenant 18,7 g de chloro-5 méthyl-4 nitro-2 aniline et 40,8 g d'imidazole. La réaction est suivie en chromatographie sur couche mince. Lorsqu'il ne reste plus de produit

de départ refroidir, ajouter de l'eau, essorer le solide obtenu. Après lavage à l'eau, le composé est repris à l'éther, au pentane, puis séché pour donner 20,6 g de (imidazolyl-1)-5 méthyl-4 nitro-2 aniline.
Point de fusion : 220° C.

Ce même produit peut être obtenu par désacétylation du composé de l'exemple 21 par la méthode décrite à l'exemple 14.

Selon le mode opératoire de l'exemple 25 les composés suivants des exemples 26 à 34 ont été préparés :

Exemple 26 **(imidazolyl-1)-5 méthyl-3 nitro-2 aniline**

**Formule (III)** A = B = T = W = C, $R_1$ = 3-$CH_3$, $R_2$ = $R_3$ = H, Y = (imidazolyl-1)-5

Point de fusion : 182° C

Exemple 27 **(imidazolyl-1)-3 nitro-2 aniline**

**Formule (III)** A = B = T = W = C, $R_1$ = $R_2$ = $R_3$ = H, Y = (imidazolyl-1)-3

Point de fusion : 160° C

Exemple 28 **Chloro-4 (imidazolyl-1)-5 nitro-2 aniline**

**Formule (III)** A = B = T = W = C, $R_1$ = 4-Cl, $R_2$ = $R_3$ = H, Y = (imidazolyl-1)-5

Point de fusion : 204° C

Exemple 29 **(imidazolyl-1)-5 méthyl-6 nitro-2 aniline**

**Formule (III)** A = B = T = W = C, $R_1$ = 6-$CH_3$, $R_2$ = $R_3$ = H, Y = (imidazolyl-1)-5

Point de fusion : 157° C

Exemple 30 **Ethyl-3 (imidazolyl-1)-5 nitro-2 aniline**

**Formule (III)** A = B = T = W = C, $R_1$ = 3-$CH_2CH_3$, $R_2$ = $R_3$ = H, Y = (imidazolyl-1)-5

Point de fusion : 142° C.

Exemple 31 **(imidazolyl-1)-5 nitro-2 trifluorométhyl-4 aniline**

**Formule (III)** A = B = T = W = C, $R_1$ = 4-$CF_3$, $R_2$ = $R_3$ = H, Y = (imidazolyl-1)-5

Point de fusion : 221° C

Exemple 32 **Chloro-4 (imidazolyl-1)-5 méthyl-3 nitro-2 aniline**

**Formule (III)** A = B = T = W = C, $R_1$ = 4-Cl, $R_2$ = 3-$CH_3$, $R_3$ = H, Y = (imidazolyl-1)-5

Point de fusion : 208° C

Exemple 33 **Diméthyl-3,4 (imidazolyl-1)-5 nitro-2 aniline**

**Formule (III)** A = B = T = W = C, $R_1$ = 4-$CH_3$, $R_2$ = 3-$CH_3$, $R_3$ = H, Y = (imidazolyl-1)-5

Point de fusion : 188° C

Exemple 34 **N-isopropyl (imidazolyl-1)-5 méthyl-4 nitro-2 aniline**

**Formule (III)** A = B = T = W = C, $R_1$ = 4-$CH_3$, $R_2$ = H, $R_3$ = iPr, Y = (imidazolyl-1)-5

Point de fusion : 159° C

Selon le mode opératoire de l'exemple 25 mais en utilisant l'imidazole substitué de façon approprié, les dérivés suivants des exemples 35 à 41 ont été préparés.

Exemple 35 **N-méthyl (méthyl-2 imidazolyl-1)-5 nitro-2 aniline**

**Formule (III)** A = B = T = W = C, $R_1$ = $R_2$ = H, $R_3$ = $CH_3$, Y = (méthyl-2 imidazolyl-1)-5

Point de fusion : 186° C

Exemple 36 **(méthyl-4 imidazolyl-1)-5 méthyl-3 nitro-2 aniline**

**Formule (III)** A = B = T = W = C, $R_1$ = 3-$CH_3$, $R_2$ = $R_3$ = H, Y = (méthyl-4 imidazolyl-1)-5

Point de fusion : 240° C

Exemple 37 **(Diméthyl-2,4 imidazolyl-1)-5 méthyl-3 nitro-2 aniline**

**Formule (III)** A = B = T = W = C, $R_1$ = 3-$CH_3$, $R_2$ = $R_3$ = H, Y = (Diméthyl-2,4 imidazolyl-1)-5

Point de fusion : 196° C

Exemple 38 **(méthyl-4 imidazolyl-1)-5 méthyl-4 nitro-2 aniline**

**Formule (III)** A = B = T = W = C, $R_1$ = 4-$CH_3$, $R_2$ = $R_3$ = H, Y = (méthyl-4 imidazolyl-1)-5

Point de fusion : 205° C

Exemple 39 **(méthyl-2 imidazolyl-1)-5 méthyl-4 nitro-2 aniline**

**Formule (III)** A = B = T = W = C, $R_1$ = 4-$CH_3$, $R_2$ = $R_3$ = H, Y = (méthyl-2 imidazolyl-1)-5

Point de fusion : 222° C

Exemple 40 **(diméthyl-2,4 imidazolyl-1)-5 methyl-4 nitro-2 aniline**

**Formule (III)** A = B = T = W = C, $R_1$ = 4-$CH_3$, $R_2$ = $R_3$ = H, Y = (diméthyl-2,4 imidazolyl-1)-5

Point de fusion : 248° C

Exemple 41 **Chloro-4 (méthyl-2 imidazolyl-1)-5 nitro-2 aniline**

**Formule (III)** A = B = T = W = C, $R_1$ = 4-Cl, $R_2$ = $R_3$ = H, Y = (méthyl-2 imidazolyl-1)-5

Point de fusion : 170° C

Exemple 42 **Chloro-4 (méthyl-4 imidazolyl-1)-5 nitro-2 aniline**

**Formule (III)** A = B = T = W = C, $R_1$ = 4-Cl, $R_2$ = $R_3$ = H, Y = (méthyl-4 imidazolyl-1)-5

Point de fusion : 220° C

Selon le mode opératoire de l'exemple 25 mais à partir de fluoro-5 nitro-2 aniline et d'imidazoles substitués ou non, les composés suivants des exemples 43 à 45 ont été préparés :

Exemple 43 **(méthyl-2 imidazolyl-1)-5 nitro-2 aniline**

**Formule (III)** A = B = T = W = C, $R_1$ = $R_2$ = $R_3$ = H, Y = (méthyl-2 imidazolyl-1)-5

Point de fusion : 229° C.

Exemple 44 **Nitro-2 (triméthyl-2,4,5 imidazolyl-1)-5 aniline**

**Formule (III)** A = B = T = W = C, $R_1$ = $R_2$ = $R_3$ = H, Y = (triméthyl-2,4,5 imidazolyl-1)-5

Point de fusion > 250° C

Exemple 45**(méthyl-4 imidazolyl-1)-5 nitro-2 aniline**

**Formule (III)** A = B = T = W = C, $R_1$ = $R_2$ = $R_3$ = H, Y = (méthyl-4 imidazolyl-1)-5

Point de fusion : 188° C

Exemple 46 **(imidazolyl-1)-5 nitro-2 aniline**

**Formule (III)** A = B = T = W = C, $R_1$ = $R_2$ = $R_3$ = H, Y = (imidazolyl-1)-5

Dans un tricol muni d'un réfrigérant et d'une ampoule à addition on place 13,7 g d'imidazole en solution dans 300 ml de diméthyl formamide. On ajoute alors par portions 10 g d'hydrure de sodium en suspension à 50 % dans l'huile. Le milieu est chauffé 1 h à 60° puis refroidi à température ambiante pour y ajouter goutte à goutte 33,3 g de fluoro-5 nitro-2 aniline en solution dans 100 ml de diméthyl formamide.

Le milieu réactionnel est alors chauffé 2 h à 70° C sous agitation puis refroidi et versé sur 1 l d'eau froide. La solution est alors extraite à l'éther, la phase organique séchée sur sulfate de magnésium et concentrée pour donner 26,3 g de (imidazolyl-1)-5 nitro-2 aniline.

Point de fusion : 185-7° C.

Exemple 47 **(benzimidazolyl-1)-5 nitro-2 aniline**

**Formule (III)** A = B = T = W = C, $R_1$ = $R_2$ = $R_3$ = H, Y = (benzimidazolyl-1)-5

Selon le mode opératoire de l'exemple 46 en faisant réagir la chloro-5 nitro-2 aniline avec le benzimidazole, on obtient la (benzimidazolyl-1)-5 nitro-2 aniline.

Point de fusion : 204-206° C.

Exemple 48 **N-méthyl (imidazolyl-1)-5 nitro-2 aniline**

**Formule (III)** A = B = T = W = C, $R_1$ = $R_2$ = H, $R_3$ = $CH_3$, Y = (imidazolyl-1)-5

Une solution de 9,3 g de N-méthyl chloro-5 nitro-2 aniline préparée selon l'exemple 9, 3,4 g d'imidazole, 5,3 g de carbonate de sodium et 50 ml de DMF est portée au reflux pendant 16 h. Laisser refroidir. Concentrer à sec puis reprendre à l'eau. Essorer, laver à l'acétonitrile, sécher pour obtenir 5,9 g de N-méthyl (imidazolyl-1)-5 nitro-2 aniline.

Point de fusion : 254° C.

Exemple 49 **(diméthyl-2,4 imidazolyl-1)-5 nitro-2 aniline**

**Formule (III)** A = B = T = W = C, $R_1$ = $R_2$ = $R_3$ = H, Y = (diméthyl-2,4 imidazolyl-1)-5

Selon l'exemple 48 la fluoro-4 nitro-2 aniline réagit avec le diméthyl-2,4 imidazole pour donner la (diméthyl-2,4 imidazolyl-1)-5 nitro-2 aniline.

Point de fusion : 194° C

Exemple 50 **[(amino-3 nitro-4 phényl)-1 méthyl-2 imidazolyl-4]-2 methyl-2 dioxolane 1,3**

**Formule (III)** A = B = T = W = C, $R_1$ = $R_2$ = $R_3$ = H, Y = [(méthyl-2 dioxolane-1,3 yl-2)-4 méthyl-2 imidazolyl-1]-5

Selon l'exemple 48, la fluoro-4 nitro-2 aniline réagit avec le (méthyl-2 imidazolyl-4)-2 méthyl-2 dioxolane-1,3, préparé à partir de l'acétyl-4 méthyl-2 imidazole et de l'éthylène glycol en présence d'acide paratoluène sulfonique, pour donner le [(amino-3 nitro-4 phényl)-1 méthyl-2 imidazolyl-4]-2 méthyl-2 dioxolane 1,3.

Point de fusion : 196° C.

Exemple 51 **amino-2 (imidazolyl-1)-6 nitro-3 pyridine**

**Formule (III)** B = T = W = C, A = N, $R_1$ = $R_2$ = $R_3$ = H, Y = (imidazolyl-1)-5

L'imidazole et l'amino-2 chloro-6 nitro-3 pyridine réagissent dans les conditions de l'exemple 48 pour donner l'amino-2 (imidazolyl-1)-6 nitro-3 pyridine.
Point de fusion : 224 ° C.

Exemple 52 **amino-4 (imidazolyl-1)-2 nitro-5 pyrimidine**

**Formule (III)** A = T = N, B = W = C, $R_1$ = $R_2$ = $R_3$ = H, Y = (imidazolyl-1)-5

La réaction de l'imidazole et de l'amino-4 chloro-2 nitro-5 pyrimidine selon l'exemple 48 conduit à l'amino-4 (imidazolyl-1)-2 nitro-5 pyrimidine
Point de fusion : 248 ° C.

Exemple 53 **N-isopropyl (imidazolyl-1)-5 nitro-2 aniline**

**Formule (III)** A = B = T = W = C, $R_1$ = $R_2$ = H, $R_3$ = iPr, Y = (imidazolyl-1)-5

A une solution de 9,4 g de N-isopropyl fluoro-5 nitro-2 aniline préparée à l'exemple 10 dans 75 ml de diméthyl formamide on ajoute par portions 4,2 g de sel de sodium de l'imidazole. Le milieu réactionnel est ensuite porté 5 h30 au reflux puis refroidi et versé dans 200 ml d'eau. Le précipité obtenu est essoré, lavé à l'eau puis dissous dans du chloroforme.

La phase organique est lavée à l'eau, séchée et concentrée pour donner 8,2 g de N-isopropyl (imidazolyl-1)-5 nitro-2 aniline.
Point de fusion : 152 ° C.

Exemple 54 **N-benzyl (imidazolyl-1)-5 nitro-2 aniline**

**Formule (III)** A = B = T = W = C, $R_1$ = $R_2$ = H, $R_3$ = $CH_2Ø$, Y = (imidazolyl-1)-5

Dans les mêmes conditions qu'à l'exemple 53, la N-benzyl fluoro-5 nitro-2 aniline obtenue à l'exemple 11 réagit avec le sel de sodium de l'imidazole pour donner la N-benzyl (imidazolyl-1)-5 nitro-2 aniline.
Point de fusion : 171 ° C.

Exemple 55 **N-méthyl (methyl-2 imidazolyl-1)-5 méthyl-4 nitro-2 aniline**

**Formule (III)** A = B = T = W = C, $R_1$ = 4-$CH_3$, $R_2$ = H, $R_3$ = $CH_3$, Y = (méthyl-2 imidazolyl-1)-5

Le N-méthyl (méthyl-2 imidazolyl-1)-5 méthyl-4 nitro-2 acétanilide préparé à l'exemple 23 est désacétylé selon le mode opératoire décrit à l'exemple 14 pour donner la N-méthyl (méthyl-2 imidazolyl-1)-5 méthyl-4 nitro-2 aniline.
Point de fusion : 155 ° C.

Exemple 56 **N-isopropyl (méthyl-2 imidazolyl-1)-5 nitro-2 aniline**

**Formule (III)** A = B = T = W = C, $R_1$ = $R_2$ = H, $R_3$ = iPr, Y = (méthyl-2 imidazolyl-1)-5

On fait réagir, comme dans le cas de l'exemple 25, la N-isopropyl fluoro-5 nitro-2 aniline préparée selon

l'exemple 10 et le méthyl-2 imidazole pour obtenir la N-isopropyl (méthyl-2 imidazolyl-1)-5 nitro-2 aniline. Point de fusion : 166° C.

Exemple 57 **Nitro-2 (triazolo-1,2,4 yl-1)-5 aniline**

**Formule (III)** A = B = T = W = C, $R_1$ = $R_2$ = $R_3$ = H,Y = (triazol-1,2,4 yl-1)-5

Suivant le mode opératoire de l'exemple 53 mais en utilisant le sel de sodium du triazole-1,2,4 et la nitro-2 fluoro-5 aniline, on obtient la nitro-2 (triazol-1,2,4 yl-1)-5 aniline.
Point de fusion : 260-265° C.

Exemple 58 **N-méthyl nitro-2 (triazol-1,2,4 yl-1)-5 aniline**

**Formule (III)** A = B = T = W = C, $R_1$ = $R_2$ = H, $R_3$ = $CH_3$, Y = (triazol-1,2,4 yl-1)-5

8,1 g de sel de sodium du triazole-1,2,4 sont chauffés au reflux 60 h dans 250 ml de DMF avec 16,7 g de N-méthyl chloro-5 nitro-2 aniline (exemple 9). Après avoir refroidi, l'insoluble est essoré et lavé au méthanol. Le DMF est concentré, le résidu est repris à chaud au méthanol et essoré.

Les 2 solides sont identiques. On obtient ainsi 12,8 g de N-méthyl nitro-2 (triazol-1,2,4 yl-1)-5 aniline.
Point de fusion : 234° C.

Exemple 59 **N-isopropyl nitro-2 (triazol-1,2,4 yl-1)-5 aniline**

**Formule (III)** A = B = T = W = C, $R_1$ = $R_2$ = H, $R_3$ = iPr, Y = (triazole-1,2,4 yl-1)-5

Comme décrit à l'exemple 53, mais en faisant réagir la N-isopropyl fluoro-5 nitro-2 aniline sur le sel de sodium du triazole 1,2,4 on obtient la N-isopropyl nitro-2 (triazole-1,2,4 yl-1)-5 aniline.
Point de fusion : 144° C.

Exemple 60 **Amino-2 (imidazolyl-1)-4 aniline**

**Formule (II)** A = B = T = W = C, $R_1$ = $R_2$ = $R_3$ = H, Y = (imidazolyl-1)-4

26,3 g de (imidazolyl-1)-5 nitro-2 aniline préparée selon l'exemple 46 sont dissous dans 150 ml d'éthanol. On ajoute alors 35 ml d'eau et 120 g de fer en poudre. Sous agitation, 2 ml d'acide chlorhydrique concentré sont ajoutés et le milieu porté 4 h au reflux. La suspension est alors filtrée à chaud sur célite. Le filtrat est refroidi et extrait au dichlorométhane. La phase organique est séchée sur sulfate de magnésium puis concentrée pour donner 17 g d'amino-2 (imidazolyl-1)-4 aniline.
Point de fusion : 168-170° C.

Selon le mode opératoire de l'exemple 60, les composés suivants des exemples 61 à 69 ont été préparés.

Exemple 61 **Amino-2 (imidazolyl-1)-4 méthyl-5 aniline**

**Formule (II)** A = B = T = W = C, $R_1$ = 5-$CH_3$, $R_2$ = $R_3$ = H, Y = (imidazolyl-1)-4

Point de fusion : 104° C.

Exemple 62 **Amino-2 (méthyl-2 imidazolyl-1)-4 méthyl-5 aniline**

**Formule (II)** A = B = T = W = C, $R_1$ = 5-$CH_3$, $R_2$ = $R_3$ = H, Y = (méthyl-2 imidazolyl-1)-4

Point de fusion : 177° C.

Exemple 63 **Amino-2 (méthyl-2 imidazolyl-1)-4 aniline**

**Formule (II)** A = B = T = W = C, $R_1$ = $R_2$ = $R_3$ = H, Y = (méthyl-2 imidazolyl-1)-4

Point de fusion : 192° C.

Exemple 64 **N-méthyl amino-2 (méthyl-2 imidazolyl-1)-4 aniline**

**Formule (II)** A = B = T = W = C, $R_1$ = $R_2$ = H, $R_3$ = $CH_3$, Y = (méthyl-2 imidazolyl-1)-4

Point de fusion : 204° C.

Exemple 65 **N-méthyl amino-2 (imidazolyl-1)-4 méthyl-5 aniline**

**Formule (II)** A = B = T = W = C, $R_1$ = 5-$CH_3$, $R_2$ = H, $R_3$ = $CH_3$, Y = (imidazolyl-1)-4

Point de fusion : 135° C.

Exemple 66 **N-méthylamino-2 (méthyl-2 imidazolyl-1)-4 méthyl-5 aniline**

**Formule (II)** A = B = T = W = C, $R_1$ = 5-$CH_3$, $R_2$ = H, $R_3$ = $CH_3$, Y = (méthyl-2 imidazolyl-1)-4

Huile utilisée brute dans l'opération ultérieure.

Exemple 67 **N-méthylamino-2 (triazol-1,2,4 yl-1)-4 aniline**

**Formule (II)** A = B = T = W = C, $R_1$ = $R_2$ = H, $R_3$ = $CH_3$, Y = (triazol-1,2,4 yl-1)-4

Point de fusion : 108° C

Exemple 68 **N-isopropylamino-2 (imidazolyl-1)-4 aniline**

**Formule (II)** A = B = T = W = C, $R_1$ = $R_2$ = H, $R_3$ = iPr, Y = (imidazolyl-1)-4

Point de fusion : 118° C

Exemple 69 **N-benzylamino-2 (imidazolyl-1)-4 aniline**

**Formule (II)** A = B = T = W = C, $R_1$ = $R_2$ = H, $R_3$-$CH_2Ø$, Y = (imidazolyl-1)-4

Point de fusion : 149° C

Exemple 70 **N-méthylamino-2 (imidazolyl-1)-4 aniline**

**Formule (II)** A = B = T = W = C, $R_1$ = $R_2$ = H, $R_3$ = $CH_3$ Y = (imidazolyl-1)-4

On hydrogène à pression normale une solution de 16,3 g de N-méthyl (imidazolyl-1)-5 nitro-2 aniline préparée à l'exemple 48 dans 900 ml de méthanol en présence de Nickel de Raney. Lorsque la quantité nécessaire d'hydrogène est absorbée, le catalyseur est éliminé par filtration et le filtrat est concentré sous vide. Reprendre à l'éther isopropylique pour obtenir 12,9 g de N-méthyl amino-2 (imidazolyl-1)-4 aniline. Point de fusion : 160° C.

Selon le mode opératoire de l'exemple 70 les composés suivants des exemples 71 à 96 ont été préparés.

Exemple 71 **Amino-2 (triazol-1,2,4 yl-1)-4 aniline**

**Formule (II)** A = B = T = W = C, $R_1$ = $R_2$ = $R_3$ = H, Y = (triazol-1,2,4 yl-1)-4

Point de fusion : 182° C

Exemple 72 **N-isopropylamino-2 (triazol-1,2,4 yl-1)-4 aniline**

**Formule (II)** A = B = T = W = C, $R_1$ = $R_2$ = H, $R_3$ = iPr, Y = (triazol-1,2,4 yl-1)-4

Point de fusion : 94° C

Exemple 73 **N-isopropylamino-2 (méthyl-2 imidazolyl-1)-4 aniline**

**Formule (II)** A = B = T = W = C, $R_1$ = $R_2$ = H, $R_3$ = iPr, Y = (méthyl-2 imidazolyl-1)-4

Point de fusion : 160° C.

Exemple 74 **N-isopropylamino-2 (imidazolyl-1)-4 méthyl-5 aniline**

**Formule (II)** A = B = T = W = C, $R_1$ = 5-$CH_3$, $R_2$ = H, $R_3$ = iPr, Y = (imidazolyl-1)-4

Point de fusion : 139° C.

Exemple 75 **Amino-2 (imidazolyl-1)-3 aniline**

**Formule (II)** A = B = T = W = C, $R_1$ = $R_2$ = $R_3$ = H, Y = (imidazolyl-1)-3

Point de fusion : 128° C.

Exemple 76 **Amino-2 (imidazolyl-1)-4 méthyl-6 aniline**

**Formule (II)** A = B = T = W = C, $R_1$ = 6-$CH_3$, $R_2$ = $R_3$ = H, Y = (imidazolyl-1)-4

Point de fusion : 152° C.

Exemple 77 **Amino-2 (imidazolyl-1)-4 éthyl-6 aniline**

**Formule (II)** A = B = T = W = C, $R_1$ = 6-$C_2H_5$, $R_2$ = $R_3$ = H, Y = (imidazolyl-1)-4

Point de fusion : 139° C.

Exemple 78 **Amino-2 chloro-5 (imidazolyl-1)-4 aniline**

**Formule (II)** A = B = T = W = C, $R_1$ = 5-Cl, $R_2$ = $R_3$ = H, Y = (imidazolyl-1)-4

Point de fusion : 164° C.

Exemple 79 **Amino-2 (imidazolyl-1)-4 méthyl-3 aniline**

**Formule (II)** A = B = T = W = C, $R_1$ = 3-$CH_3$, $R_2$ = $R_3$ = H, Y = (imidazolyl-1)-4

Point de fusion : 152° C.

Exemple 80 **Amino-2 (imidazolyl-1)-4 trifluorométhyl-5 aniline**

**Formule (II)** A = B = T = W = C, $R_1$ = 5-$CF_3$, $R_2$ = $R_3$ = H, Y = (imidazolyl-1)-4

Point de fusion : 186° C.

Exemple 81 **Amino-2 chloro-5 (imidazolyl-1)-4 méthyl-6 aniline**

**Formule (II)** A = B = T = W = C, $R_1$ = 5-Cl, $R_2$ = 6-$CH_3$, $R_3$ = H, Y = (imidazolyl-1)-4

Point de fusion : 131° C.

Exemple 82 **Amino-2 diméthyl-5,6 (imidazolyl-1)-4 aniline**

**Formule (II)** A = B = T = W = C, $R_1$ = 5-$CH_3$, $R_2$ = 6-$CH_3$, $R_3$ = H, Y = (imidazolyl-1)-4

Point de fusion : 132° C.

Exemple 83 **Amino-2 (méthyl-4 imidazolyl-1)-4 méthyl-6 aniline**

**Formule (II)** A = B = T = W = C, $R_1$ = 6-$CH_3$, $R_2$ = $R_3$ = H, Y = (méthyl-4 imidazolyl-1)-4

Point de fusion : 177 °C.

Exemple 84 **Amino-2 (diméthyl-2,4 imidazolyl-1)-4 methyl-6 aniline**

**Formule (II)** A = B = T = W = C, $R_1$ = 6-$CH_3$, $R_2$ = $R_3$ = H, Y = (diméthyl-2,4 imidazolyl-1)-4

Point de fusion : 178 °C.

Exemple 85 **Amino-2 (méthyl-4 imidazolyl-1)-4 méthyl-5 aniline**

**Formule (II)** A = B = T = W = C, $R_1$ = 5-$CH_3$, $R_2$ = $R_3$ = H, Y = (méthyl-4 imidazolyl-1)-4

Point de fusion : 146 °C.

Exemple 86 **Amino-2 (méthyl-2 imidazolyl-1)-4 méthyl-5 aniline**

**Formule (II)** A = B = T = W = C, $R_1$ = 5-$CH_3$, $R_2$ = $R_3$ = H, Y = (méthyl-2 imidazolyl-1)-4

Point de fusion : 172 °C.

Exemple 87 **Amino-2 (diméthyl-2,4 imidazolyl-1)-4 méthyl-5 aniline**

**Formule (II)** A = B = T = W = C, $R_1$ = 5-$CH_3$, $R_2$ = $R_3$ = H, Y = (diméthyl-2,4 imidazolyl-1)-4

Huile utilisée brute à l'étape suivante.

Exemple 88 **Amino-2 chloro-5 (méthyl-2 imidazolyl-1)-4 aniline**

**Formule (II)** A = B = T = W = C, $R_1$ = 5-Cl, $R_2$ = $R_3$ = H, Y = (méthyl-2 imidazolyl-1)-4

Point de fusion : 191 °C.

Exemple 89 **Amino-2 chloro-5 (méthyl-4 imidazolyl-1)-4 aniline**

**Formule (II)** A = B = T = W = C, $R_1$ = 5-Cl, $R_2$ = $R_3$ = H, Y = (méthyl-4 imidazolyl-1)-4

Point de fusion : 168 °C.

Exemple 90 **Amino-2 (triméthyl-2,4,5 imidazolyl-1)-4 aniline**

**Formule (II)** A = B = T = W = C, $R_1$ = $R_2$ = $R_3$ = H, Y = (triméthyl-2,4,5 imidazolyl-1)-4

Point de fusion : 194° C.

Exemple 91 **Amino-2 (méthyl-4 imidazolyl-1)-4 aniline**

**Formule (II)** A = B = T = W = C, $R_1 = R_2 = R_3$ = H, Y = (méthyl-4 imidazolyl-1)-4

Point de fusion : 153° C.

Exemple 92 **Amino-2 (benzimidazolyl-1)-4 aniline**

**Formule (II)** A = B = T = W = C, $R_1 = R_2 = R_3$ = H, Y = (benzimidazolyl-1)-4

Point de fusion : 175° C.

Exemple 93 **Amino-2 (diméthyl-2,4 imidazolyl-1)-4 aniline**

**Formule (II)** A = B = T = W = C, $R_1 = R_2 = R_3$ = H, Y = (diméthyl-2,4 imidazolyl-1)-4

Point de fusion : 133° C.

Exemple 94 **[(Diamino-3,4 phényl)-1 méthyl-2 imidazolyl-4]-2 méthyl-2 dioxolane-1,3**

**Formule (II)** A = B = T = W = C, $R_1 = R_2 = R_3$ = H, Y = [(méthyl-2 dioxolane-1,3 yl-2)-4 méthyl-2 imidazolyl-1]-4

Point de fusion : 206° C.

Exemple 95 **Diamino-2,3 (imidazolyl-1)-6 pyridine**

**Formule (II)** A = N, B = T = W = C, $R_1 = R_2 = R_3$ = H, Y = (imidazolyl-1)-4

Point de fusion : 186° C.

Exemple 96 **Diamino-4,5 (imidazolyl-1)-2 pyrimidine**

**Formule (II)** A = T = N, B = W = C, $R_1 = R_2 = R_3$ = H, Y = (imidazolyl-1)-4

Point de fusion : 240° C (décomposition).

Exemple 97 **[(imidazolyl-2) thioacétyl]-5 chloro-2 nitrobenzène**

**Formule (X)** $R_1 = R_2 = R_8$ = H, $R_7$ = imidazolyl-2, X = Cl

Une solution de 14,4 g de (α bromo acétyl)-5 chloro-2 nitro benzène, et 5,2 g de mercapto-2 imidazole dans 310 ml de méthanol est agitée pendant une heure à température ambiante. Concentrer sous vide,

reprendre à l'éther, essorer pour obtenir 19 g de bromhydrate de [(imidazolyl-2)thioacétyl]-5 chloro-2 nitrobenzène. Point de fusion: 240-242 °C.

Ajouter les 19 g de solide obtenus précédemment à une solution de 5,5 g de carbonate de sodium dans 100 ml d'eau. Agiter 1 heure, essorer, laver à l'eau puis à l'alcool isopropylique et à l'éther isopropylique pour obtenir 13,9 g de [(imidazolyl-2) thioacetyl]-5 chloro-2 nitrobenzène.
Point de fusion : 160 °C

Exemple 98 **(chloro-4 nitro-3 phényl)-3 imidazo[2,1-b]thiazole**

**Formule (IX)** A = T = C, $R_1$ = $R_2$ = H, X = Cl, Y = imidazo [2,1-b]thiazolyl-3

A 1400g de PPA maintenu à 110 °C on ajoute 38,6 g de [(imidazolyl-2) thioacétyl]-5 chloro-2 nitrobenzène préparés précédemment. Porter le mélange à 120 °C pendant 3 h puis laisser une nuit à température ambiante. Verser sur un mélange d'eau et de glace.

Ramener le pH à 7 par addition d'une solution d'ammoniaque. Essorer le précipité obtenu, laver à l'eau puis à l'éther isopropylique et à l'acétone pour obtenir 41,1 g de (chloro-4 nitro-3 phényl)-3 imidazo [2,1-b] thiazole.
Point de fusion : 206 °C.

Exemple 99 **Dihydro-5,6 (chloro-4 nitro-3 phényl)-3 imidazo [2,1-b] thiazole**

**Formule (IX)** A = T = C, $R_1$ = $R_2$ = H, X = Cl, Y = (dihydro-5,6 imidazo[2,1-b] thiazolyl)-3

10,4 g de (α bromoacétyl)-5 chloro-2 nitrobenzène et 3,8 g d'imidazolidine thione-2 dans 200 ml de méthanol sont mis en réaction pendant une heure à température ambiante. Le bromhydrate intermédiaire (point de fusion 190 °C) est neutralisé comme à l'exemple 97 pour conduire à 10,5 g de tétrahydro-2,3,5,6 hydroxy-3 (chloro-4 nitro-3 phényl)-3 imidazo [2,1-b] thiazole (point de fusion 187 °C) dont le traitement dans 200 g de PPA selon l'exemple 98 permet d'obtenir 8,7 g de dihydro-5,6 (chloro-4 nitro-3 phényl)-3 imidazo [2,1-b] thiazole.
Point de fusion : 174 °C.

Exemple 100 **Amino-2 (imidazo [2,1-b] thiazolyl-3)-4 aniline**

**Formule (II)** A = B = T = W = C, $R_1$ = $R_2$ = $R_3$ = H, Y = (imidazo [2,1-b] thiazolyl-3)-4

Dans un autoclave de 1 l contenant 500 ml de méthoxy-2 éthanol froid, on dissout 55 g d'ammoniac. Rajouter alors 41,1 g de (chloro-4 nitro-3 phényl)-3 imidazo [2,1-b] thiazole préparé à l'exemple 98 et porter 24 h à 150 °C sous pression. Laisser refroidir. Evaporer le solvant, reprendre à l'eau, filtrer pour obtenir 30 g de nitro-2 (imidazo [2,1-b] thiazolyl-3)-4 aniline.
Point de fusion : 266 °C.

15,6 g de ce composé sont soumis sans purification ultérieure à une hydrogénation en présence de Nickel de Raney selon l'exemple 70 pour donner 6,5 g d'amino-2 (imidazo [2,1-b] thiazolyl-3)-4 aniline.
Point de fusion : 210 °C

Exemple 101 **(αChloro acétyl)-5 benzimidazolone-2**

**Formule (XII)** $R_1$ = $R_2$ = $R_3$ = $R_8$ = H, X = Cl

26,8 g de benzimidazolone sont ajoutés par portions et à une température inférieure à 5 °C, sur un mélange de 80 g de chlorure d'aluminium, 34 ml de chlorure de chloroacétyle et 100 ml de chlorure de

méthylène anhydre. Après la fin de l'addition le mélange réactionnel est porté lentement au reflux et maintenu à cette température pendant 3 h. Puis il est refroidi et versé sur un mélange glace/acide chlorhydrique. Le précipité obtenu est essoré, lavé à l'eau jusqu'à neutralité, puis à l'alcool éthylique et enfin à l'éther.

Après séchage, on obtient 35,1 g de (α chloroacétyl)-5 benzimidazolone-2.

Point de fusion 281-286 °C. (décomp.)

Selon le mode opératoire de l'exemple 101, les composés suivants des exemples 102, 103 ont été préparés.

Exemple 102 **(chloro-2 propionyl)-5 benzimidazolone-2**

**Formule (XII)** $R_1 = R_2 = R_3 = H$, $X = Cl$, $R_8 = CH_3$

Point de fusion : 270-280 °C (décomposition).

Exemple 103 **(α chloroacétyl)-5 méthyl-6 benzimidazolone-2**

**Formule (XII)** $R_1 = 6\text{-}CH_3$ $R_2 = R_3 = R_8 H$, $X = Cl$

Point de fusion : 263-266 °C (décomposition).

Exemple 104 **Chlorhydrate de [(imidazolyl-2) thioacétyl]-5 benzimidazolone-2**

**Formule (XI)** $R_1 = R_2 = R_3 = R_8 = H$, $R_7$ = imidazolyl-2

13,8 g de (α chloro acétyl)-5 benzimidazolone-2 préparés à l'exemple 101 et 6,6 g de mercapto-2 imidazole dans 75 ml de N-méthyl pyrrolidone sont portés à 100° pendant 3 h. Les cristaux obtenus sont essorés à froid, lavés à l'acétone et à l'éther, puis séchés.

On obtient ainsi 19,9 g de chlorhydrate de [ (imidazolyl-2) thioacétyl]-5 benzimidazolone-2.

Point de fusion : 194 °C.

Exemple 105 **Fumarate de [(dihydro-5,6 imidazo [2,1-b] thiazolyl)-3]-5 benzimidazolone-2**

**Formule (I)** A = B = T = W = C, $R_1 = R_2 = R_3 = H$, Y = [(dihydro-5,6 imidazo [2,1-b] thiazolyl)-3]-5, Z = OH

Une solution de 9 g d'imidazolidinéthione-2, 22,5 g de (αchloroacétyl)-5 benzimidazolone-2 préparé à l'exemple 101, dans 450 ml de N-méthyl pyrrolidone est portée à 150° pendant 3 h. Le précipité obtenu est essoré, lavé à l'acétone et à l'éther, puis dissous dans 500 ml d'eau.

La solution ainsi obtenue est filtrée avec passage au noir, puis alcalinisée à froid par de l'ammoniaque concentrée, et le précipité formé est essoré, lavé à l'eau jusqu'à neutralité, puis à l'acétone, et séché. On obtient ainsi 16,8 g de [(dihydro-5,6 imidazo [2,1-b] thiazolyl)-3]-5 benzimidazolone-2 sous forme base, qui sont dissous à chaud dans 750 ml de méthanol. A cette solution chaude on ajoute 11,3 g (1,5 éq) d'acide fumarique dans 100 ml de méthanol, puis on filtre à chaud avec passage au noir. La solution est ensuite concentrée de moitié. Le produit cristallise à froid sous forme de 3/2 fumarate. On obtient ainsi 7,5 g de 3/2 fumarate de [(dihydro-5,6 imidazo [2,1-b] thiazolyl)-3]-5 benzimidazolone-2.

Point de fusion : 211-213 °C.

Selon le mode opératoire de l'exemple 105, les composés suivants des exemples 106, 107 ont été préparés.

Exemple 106 **Chlorhydrate de [(dihydro-5,6 méthyl-2 imidazo[ 2,i-b] thiazolyl)-3]-5 benzimidazolone-2**

**Formule (I)** A = B = T = W = C, $R_1 = R_2 = R_3 = H$, Y = (dihydro-5,6 méthyl-2 imidazo [2,1-b] thiazolyl)-3]-5, Z = OH

Point de fusion : 329-330 ° C (décomposition).

Exemple 107 **Chlorhydrate de [(dihydro-5,6 imidazo [2,1-b] thiazolyl)-3]-5 méthyl-6 benzimidazolone-2**

**Formule (I)** A = B = T = W = C, $R_1$ = 6-$CH_3$, $R_2 = R_3 = H$, Y = (dihydro-5,6 imidazo [2,1-b] thiazolyl)-3]-5, Z = OH

Point de fusion : 340-345 ° C (décomposition).

Exemple 108 **[(imidazo [2,1-b] thiazolyl)-3]-5 benzimidazolone-2**

**Formule (I)** A = B = T = W = C, $R_1 = R_2 = R_3 = H$, Y = [(imidazo [2,1-b] thiazolyl)-3]-5, Z = OH

19,9 g de chlorhydrate de [(imidazolyl-2) thioacétyl]-5 benzimidazolone-2 préparés à l'exemple 104 et 600 g de PPA sont portés à 90 ° pendant 8 h. Le mélange encore chaud est versé lentement sur 5 l d'eau/glace. La solution obtenue est alcalinisée par de la lessive de soude, à froid. Il se forme un précipité abondant qui est essoré et lavé à l'eau. Ce précipité est repris par 250 ml de DMF au reflux. L'insoluble est éliminé et la solution de DMF est concentrée, après passage au noir, jusqu'à début de cristallisation. Les cristaux obtenus sont essorés, lavés à l'eau, à l'acétone et à l'éther, puis séchés.

On obtient ainsi 6,2 g de [(imidazo [2,1-b] thiazolyl)-3]-5 benzimidazolone-2.
Point de fusion > 330 ° C (décomposition).

Exemple 109 **(imidazolyl-1)-5 méthyl-6 benzimidazolone-2**

**Formule (I)** A = B = T = W = C, $R_1$ = 6-$CH_3$, $R_2 = R_3 = H$, Y = (imidazolyl-1)-5, Z = OH

4,8 g d'amino-2 (imidazolyl-1)-4 méthyl-5 aniline préparée à l'exemple 61 sont mélangés intimement avec 2,3 g d'urée finement broyée. Le mélange est chauffé à 180 ° C pendant 2 h 30. On observe la fusion du milieu avec dégagement de l'ammoniac formé puis prise en masse. Après refroidissement, le solide est dissous dans 50 ml de soude 1N. La solution est extraite à l'éther puis passée au noir et filtrée. Le filtrat est acidifié à pH 7 avec de l'acide chlorhydrique dilué. Un précipité se forme. Il est essoré, lavé à l'eau puis à l'acétone.

On obtient ainsi 3,7 g de (imidazolyl-1)-5 méthyl-6 benzimidazolone-2.
Point de fusion : > 340 ° C.

Selon le mode opératoire de l'exemple 109, les composés suivants des exemples 110 à 122 ont été obtenus :

Exemple 110 **Chlorhydrate de (imidazolyl-1)-5 benzimidazolone-2**

**Formule (I)** A = B = T = W = C, $R_1 = R_2 = R_3 = H$, Y = (imidazolyl-1)-5, Z = OH

Point de fusion : > 330 ° C

Exemple 111 **(Imidazolyl-1)-5 méthyl-3 benzimidazolone-2**

**Formule (I)** A = B = T = W = C, $R_1$ = $R_2$ = H, $R_3$ = $CH_3$, Y = (imidazolyl-1)-5, Z = OH

Point de fusion : 254° C

Exemple 112 **Chlorhydrate de (méthyl-2 imidazolyl-1)-5 méthyl-6 benzimidazolone-2**

**Formule (I)** A = B = T = W = C, $R_1$ = 6-$CH_3$, $R_2$ = $R_3$ = H, Y = (méthyl-2 imidazolyl-1)-5, Z = OH

Point de fusion > 340° C

Exemple 113 **Chlorhydrate de (méthyl-2 imidazolyl-1)-5 benzimidazolone-2**

**Formule (I)** A = B = T = W = C, $R_1$ = $R_2$ = $R_3$ = H, Y = (méthyl-2 imidazolyl-1)-5, Z = OH

Point de fusion : 286-288° C

Exemple 114 **Chlorhydrate de (méthyl-2 imidazolyl-1)-5 méthyl-3 benzimidazolone-2**

**Formule (I)** A = B = T = W = C, $R_1$ = $R_2$ = H, $R_3$ = $CH_3$, Y = (méthyl-2 imidazolyl-1)-5, Z = OH

Point de fusion du chlorhydrate monohydraté : 315° C

Exemple 115 **Chlorhydrate de diméthyl-3,6 (imidazolyl-1)-5 benzimidazolone-2**

**Formule (I)** A = B = T = W = C, $R_1$ = 6-$CH_3$, $R_2$ = H, $R_3$ = $CH_3$, Y = (imidazolyl-1)-5, Z = OH

Point de fusion du chlorhydrate monohydraté : 335-340° C.

Exemple 116 **Chlorhydrate de diméthyl-3,6 (méthyl-2 imidazolyl-1)-5 benzimidazolone-2**

**Formule (I)** A = B = T = W = C, $R_1$ = 6-$CH_3$, $R_2$ = H, $R_3$ = $CH_3$, Y = (méthyl-2 imidazolyl-1)-5, Z = OH

Point de fusion du chlorhydrate monohydraté : >340° C.

Exemple 117 **Chlorhydrate de méthyl-3 (triazol-1,2,4 yl-1)-5 benzimidazolone-2**

**Formule (I)** A = B = T = W = C, $R_1$ = $R_2$ = H, $R_3$ = $CH_3$, Y = (triazol-1,2,4 yl-1)-5, Z = OH

Point de fusion : 238-241° C.

Exemple 118 **Chlorhydrate de isopropyl-3 (imidazolyl-1)-5 benzimidazolone-2**

**Formule (I)** A = B = T = W = C, $R_1$ = $R_2$ = H, $R_3$ = iPr, Y = (imidazolyl-1)-5, Z = OH

Point de fusion : 260-265° C.

Exemple 119 **Benzyl-3 (imidazolyl-1)-5 benzimidazolone-2**

**Formule (I)** A = B = T = W = C, $R_1$ = $R_2$ = H, $R_3$ = CH2-Ø, Y = (imidazolyl-1)-5, Z = OH

Point de fusion : 289-293° C.

Exemple 120 **(Triazol-1,2,4 yl-1)-5 benzimidazolone-2**

**Formule (I)** A = B = T = W = C, $R_1$ = $R_2$ = $R_3$ = H, Y = (triazol-1,2,4 yl-1)-5, Z = OH

Point de fusion : 360° C.

Exemple 121 **Isopropyl-3 (imidazolyl-1)-5 méthyl-6 benzimidazolone-2**

**Formule (I)** A = B = T = W = C, $R_1$ = 6-$CH_3$, $R_2$ = H, $R_3$ = iPr, Y = (imidazolyl-1)-5, Z = OH

Point de fusion : 268° C.

Exemple 122 (imidazolyl-1)-5 **imidazo [4,5-b] pyridinone-2**

**Formule (I)** A = N, B = T = W = C, $R_1$ = $R_2$ = $R_3$ = H, Y = (imidazolyl-1)-5, Z = OH

Point de fusion : 331° C.

Exemple 123 **(imidazolyl-1)-5 benzimidazole thione-2**

**Formule (I)** $R_1$ = $R_2$ = $R_3$ = H, Y = (imidazolyl-1)-5, Z = SH

Dissoudre 20,4 g d'amino-2 (imidazolyl-1)-4 aniline préparée à l'exemple 60 dans 800 ml d'éthanol. Ajouter goutte à goutte 9 ml de disulfure de carbone. Porter au reflux pendant 6 h. Refroidir. Filtrer le précipité puis le laver à l'éthanol. La recristallisation dans le diméthylformamide permet d'obtenir 14,5 g de (imidazolyl-1)-5 benzimidazole thione-2.

Point de fusion : 347° C.

Selon le mode opératoire de l'exemple 123, les composés suivants des exemples 124 à 127 ont été préparés.

Exemple 124 **(imidazolyl-1)-5 méthyl-3 benzimidazole thione-2**

**Formule (I)** A = B = T = W = C, $R_1$ = $R_2$ = H, $R_3$ = $CH_3$, Y = (imidazolyl-1)-5, Z = SH

Point de fusion : 295° C.

Exemple 125 **(Triazol-1,2,4 yl-1)-5 benzimidazole thione-2**

**Formule (I)** A = B = T = W = C, $R_1$ = $R_2$ = $R_3$ = H, Y = (triazol-1,2,4 yl-1)-5, Z = SH

Point de fusion : 335° C.

Exemple 126 **(Imidazolyl-1)-5 isopropyl-3 méthyl-6 benzimidazole thione-2**

**Formule (I)** A = B = T = W = C, $R_1$ = 6-$CH_3$, $R_2$ = H, $R_3$ = iPr, Y = (imidazolyl-1)-5, Z = SH

Point de fusion : 277° C.

Exemple 127 **(Imidazolyl-1)-5 isopropyl-3 benzimidazole thione-2**

**Formule (I)** A = B = T = W = C, $R_1$ = $R_2$ = H, $R_3$ = iPr, Y = (imidazolyl-1)-5, Z = SH

Point de fusion 281° C.

Exemple 128 **(Imidazolyl-1)-5 (méthylthio)-2 benzimidazole**

**Formule (I)** A = B = T = W = C, $R_1$ = $R_2$ = $R_3$ = H, Y = (imidazolyl-1)-5, Z = $SCH_3$

A 11 g de (imidazolyl-1)-5 benzimidazole thione-2 obtenue à l'exemple 123 en suspension dans 150 ml d'éthanol, on ajoute 10 ml de NaOH 5N. Agiter 30 mn pour obtenir la dissolution totale. Additionner goutte à goutte 8,5 g de iodure de méthyle dans 30 ml d'éthanol. Laisser la réaction à température ambiante pendant 1 nuit. Concentrer sous vide, reprendre au chloroforme, décanter. La phase organique ainsi obtenue est lavée à l'eau, séchée puis concentrée sous vide pour donner une huile qui traitée à l'acétate d'éthyle/pentane conduit à des cristaux. La recristallisation dans l'acétonitrile permet d'obtenir 7,3 g d'-(imidazolyl-1)-5 (méthylthio)-2 benzimidazole.

Point de fusion : 153° C.

Les composés suivants des exemples 129 à 132 ont été obtenus selon le mode opératoire de l'exemple 128.

Exemple 129 **(imidazolyl-1)-5 (allylthio)-2 benzimidazole**

**Formule (I)** A = B = T = W = C, $R_1$ = $R_2$ = $R_3$ = H, Y = (imidazolyl-1)-5, Z = allylthio

Point de fusion : 114° C.

Exemple 130 **(Imidazolyl-1)-5 (propargylthio)-2 benzimidazole**

**Formule (I)** A = B = T = W = C, $R_1$ = $R_2$ = $R_3$ = H, Y = (imidazolyl-1)-5, Z = propargylthio

Point de fusion : 246° C.

Exemple 131 **Isopropyl-3 (propargylthio)-2 (triazol-1,2,4 yl-1)-5 benzimidazole**

**Formule (I)** A = B = T = W = C, $R_1$ = $R_2$ = H, $R_3$ = iPr, Y = (triazol-1,2,4 yl-)-5, Z = propargylthio

Point de fusion : 100 ° C.

Exemple 132 **(méthyl-2 imidazolyl-1)-5 isopropyl-3 (propargylthio)-2 benzimidazole**

**Formule (I)** A = B = T = W = C, $R_1$ = $R_2$ = H, $R_3$ = iPr, Y = (méthyl-2 imidazolyl-1)-5, Z = propargylthio

Point de fusion : 159 ° C.

Exemple 133 **(imidazolyl-1)-5 (méthylsulfinyl)-2 benzimidazole**

**Formule (I)** A = B = T = W = C, $R_1$ = $R_2$ = $R_3$ = H, Y = (imidazolyl-1)-5, Z = $SOCH_3$

A une solution refroidie à 0 ° C de 4,3g d'(imidazolyl-1)-5 (méthylthio)-2 benzimidazole obtenu à l'exemple 128 dans 200 ml d'une solution méthanol/chloroforme (50/50), on ajoute goutte à goutte 4,2 g d'acide métachloro perbenzoïque dans 40 ml de $CHCl_3$. Après 20 mn de réaction, laisser remonter la température à l'ambiante. Concentrer, reprendre avec une solution saturée de bicarbonate de sodium. Puis extraire au dichlorométhane. La phase organique séchée, concentrée permet d'obtenir après traitement à l'acétonitrile 3,5 g d'(imidazolyl-1)-5 (méthylsulfinyl)-2 benzimidazole.
Point de fusion : 214 ° C.

Exemple 134 **(Pyridyl-4 amino)-2 (imidazolyl-1)-5 benzimidazole**

**Formule (I)** A = B = T = W = C, $R_1$ = $R_2$ = $R_3$ = H, Y = (imidazolyl-1)-5, Z = (pyridyl-4 amino)

Dans une solution de 5,2 g d'amino-2 (imidazolyl-1)-4 aniline préparée à l'exemple 60 et de 6,6 g d'oxyde de mercure (II) rouge dans 60 ml de diméthyl formamide, on introduit goutte à goutte une solution de 5,5 g de S-méthyl pyridyl-4 dithiocarbamate dans 40 ml de diméthyl formamide. Agiter 3 h à température ambiante. Verser la solution dans 1800 ml d'acide chlorhydrique à 3 % et chauffer au reflux pendant 1/4 h. Filtrer à chaud, refroidir. Ramener le pH à 8 avec une solution d'ammoniaque. Le précipité ainsi obtenu est repris dans de l'éthanol à chaud, traité au charbon actif. Un passage acide-base permet d'obtenir 1,8 g de (pyridyl-4 amino)-2 (imidazolyl-1)-5 benzimidazole.
Point de fusion : 180 ° C.

Exemple 135 **(Imidazolyl-1)-5 (diméthoxy-2,4 phényl)-2 benzimidazole**

**Formule (I)** A = B = T = W = C, $R_1$ = $R_2$ = $R_3$ = H, Y = (imidazolyl-1)-5, Z = (diméthoxy-2,4 phényl)

Dissoudre 9,1 g d'amino-2 (imidazolyl-1)-4 aniline préparée à l'exemple 60 dans 150 ml de méthanol. Ajouter 8,6 g de diméthoxy-2,4 benzaldéhyde. Porter au reflux 13 h. Concentrer sous vide. Reprendre au dichlorométhane/acétate d'éthyle pour obtenir un précipité dont la purification sur gel de silice conduit à 2 g de (imidazolyl-1)-5 (diméthoxy-2,4 phényl)-2 benzimidazole.
Point de fusion : 188 ° C.

Exemple 136 **(Imidazolyl-1)-5 (méthoxy-2 méthylthio-4 phényl)-2 benzimidazole**

**Formule (I)** A = B = T = W = C, $R_1$ = $R_2$ = $R_3$ = H, Y = (imidazolyl-1)-5, Z = méthoxy-2 méthylthio-4 phényl

A 200 ml de $POCl_3$ on ajoute par portions le mélange de 8,7 g d'amino-2 (imidazolyl-1)-4 aniline (exemple 60) et 9,9 g d'acide (méthoxy-2 méthylthio-4) benzoïque. Porter au reflux 4 h. Le solide noir obtenu est filtré et lavé à l'éther. Mettre les cristaux dans 200 ml d'acide chlorhydrique 1N. Filtrer, alcaliniser les eaux mères par NaOH 30 % et extraire au chloroforme. Sécher, concentrer. Après purification partielle sur gel de silice, une recristallisation dans l'acétonitrile conduit à 2,1 g de (imidazolyl-1)-5 (méthoxy-2 méthylthio-4 phényl)-2 benzimidazole.

Point de fusion : 182° C.

Exemple 137 **(Imidazolyl-1)-5 (méthoxy-2 méthylsulfinyl-4 phényl)-2 benzimidazole**

**Formule (I)** A = B = T = W = C, $R_1$ = $R_2$ = $R_3$ = H, Y = (imidazolyl-1)-5, Z = (méthoxy-2 méthylsulfinyl-4 phényl)

A une solution de 5,8 g d'(imidazolyl-1)-5 (méthoxy-2 méthylthio-4 phényl)-2 benzimidazole préparé à l'exemple 136 dans 60 ml de $CHCl_3$, on ajoute 3,6 g d'acide méta chloro perbenzoïque par petites portions et en maintenant la température inférieure à -30° C. Agiter 1 h à -30° C puis laisser remonter la température à l'ambiante. Ajouter une solution saturée de $NaHCO_3$. Extraire au chloroforme. Sécher, concentrer. Reprendre le produit dans l'acétonitrile pour obtenir 2,8 g d'(imidazolyl-1)-5 (méthoxy-2 méthylsulfinyl-4 phényl)-2 benzimidazole.

Point de fusion : 208° C.

Exemple 138 **(Imidazolyl-1)-5 (pyridyl-4)-2 benzimidazole**

**Formule (I)** A = B = T = W = C, $R_1$ = $R_2$ = $R_3$ = H, Y = (imidazolyl-1)-5, Z = pyridyl-4

A une solution de 15,3 g d'amino-2 (imidazolyl-1)-4 aniline (exemple 60) dans 250 ml de méthanol on ajoute 9,4 g de formyl-4 pyridine. Porter au reflux pendant 8 h. Concentrer sous vide, puis reprendre le produit brut dans 70 ml de nitrobenzène et porter à 180° C pendant 4 h. Après distillation du solvant, le solide obtenu est purifié partiellement sur gel de silice puis recristallisé dans du méthanol pour donner 4,5 g d'(imidazolyl-1)-5 (pyridyl-4)-2 benzimidazole.

Point de fusion : 265° C.

Selon le mode opératoire de l'exemple 138, les composés suivants des exemples 139 à 145 ont pu être préparés.

Exemple 139 **(Imidazolyl-1)-5 méthyl-6 (pyridyl-4)-2 benzimidazole**

**Formule (I)** A = B = T = W = C, $R_1$ = 6-$CH_3$ $R_2$ = $R_3$ = H, Y = (imidazolyl-1)-5, Z = pyridyl-4

Point de fusion : 288° C.

Exemple 140 **(Imidazolyl-1)-5 (pyridyl-2)-2 benzimidazole**

**Formule (I)** A = B = T = W = C, $R_1$ = $R_2$ = $R_3$ = H, Y = (imidazolyl-1)-5, Z = pyridyl-2

Point de fusion : 219° C.

Exemple 141 **(Imidazolyl-1)-5 (pyridyl-3)-2 benzimidazole**

**Formule (I)** A = B = T = W = C, $R_1$ = $R_2$ = $R_3$ = H, Y = (imidazolyl-1)-5, Z = pyridyl-3

Point de fusion : 250° C.

Exemple 142 **(Pyridyl-4)-2 (triazol-1,2,4 yl-1)-5 benzimidazole**

**Formule (I)**A = B = T = W = C, $R_1$ = $R_2$ = $R_3$ = H, Y = (triazol-1,2,4 yl-1)-5, Z = pyridyl-4

Point de fusion : 248° C

Exemple 143 **(imidazolyl-1)-5 (méthoxy-2 pyridyl-3)-2 benzimidazole**

**Formule (I)** A = B = T = W = C, $R_1$ = $R_2$ = $R_3$ = H, Y = (imidazolyl-1)-5, Z = (méthoxy-2 pyridyl-3)

Point de fusion : 242° C

Exemple 144 **(méthyl-2 imidazolyl-1)-5 (pyridyl-4)-2 benzimidazole**

**Formule (I)** A = B = T = W = C, $R_1$ = $R_2$ = $R_3$ = H, Y = (méthyl-2 imidazolyl-1)-5, Z = pyridyl-4

Point de fusion : 295° C

Exemple 145 **[(Imidazo [2,1-b] thiazolyl)-3]-5 (pyridyl-4)-2 benzimidazole**

**Formule (I)** A = B = T = W = C, $R_1$ = $R_2$ = $R_3$ = H, Y = [(imidazo [2,1-b] thiazolyl)-3]-5, Z = pyridyl-4

Point de fusion : 332° C

Exemple 146 **(Pyridyl-4)-2 (triméthyl-2,4,5 imidazolyl-1)-5 benzimidazole**

**Formule (I)** A = B = T = W = C, $R_1$ = $R_2$ = $R_3$ = H, Y = (triméthyl-2,4,5 imidazolyl-1)-5, Z = pyridyl-4

Point de fusion : 278° C

Exemple 147 **(Méthyl-4 imidazolyl-1)-5 (pyridyl-4)-2 benzimidazole**

**Formule (I)** A = B = T = W = C, $R_1$ = $R_2$ = $R_3$ = H, Y = (méthyl-4 imidazolyl-1)-5, Z = pyridyl-4

Point de fusion : 264° C

Exemple 148 **(Diméthyl-2,4 imidazolyl-1)-5 (pyridyl-4)-2 benzimidazole**

**Formule (I)** A = B = T = W = C, $R_1$ = $R_2$ = $R_3$ = H, Y = (diméthyl-2,4 imidazolyl-1)-5, Z = pyridyl-4

Point de fusion : 291° C

Exemple 149 **(benzimidazolyl-1)-5 (pyridyl-4)-2 benzimidazole**

**Formule (I)** A = B = T = W = C, $R_1$ $R_2$ = $R_3$ = H, Y = (benzimidazolyl-1)-5, Z = pyridyl-4

Point de fusion : 280-282 °C

Exemple 150 **Chloro-6 (imidazolyl-1)-5 (pyridyl-4)-2 benzimidazole**

**Formule (I)** A = B = T = W = C, $R_1$ = 6-Cl, $R_2$ = $R_3$ = H, Y = (imidazolyl-1)-5, Z = pyridyl-4

Point de fusion : 326 °C

Exemple 151 **[(imidazolyl-1)-5 benzimidazolyl-2]-4 acétanilide**

**Formule (I)**A = B = T = W = C, $R_1$ = $R_2$ = $R_3$ = H, Y = (imidazolyl-1)-5, Z = N acétyl amino-4 phényl

Point de fusion : 362 °C

Exemple 152 **(imidazolyl-1)-5 méthyl-7 (pyridyl-4)-2 benzimidazole**

**Formule (I)** A = B = T = W = C, $R_1$ = 7-$CH_3$, $R_2$ = $R_3$ = H, Y = (imidazolyl-1)-5, Z = pyridyl-4

Point de fusion : 289 °C

Exemple 153 **méthyl-7 (méthyl-4 imidazolyl-1)-5 (pyridyl-4)-2 benzimidazole**

**Formule (I)** A = B = T = W = C, $R_1$ = 7-$CH_3$, $R_2$ = $R_3$ = H, Y = (méthyl-4 imidazolyl-1)-5, Z = pyridyl-4

Point de fusion : 285 °C

Exemple 154 **(Diméthyl-2,4 imidazolyl-1)-5 méthyl-7 (pyridyl-4)-2 benzimidazole**

**Formule (I)** A = B = T = W = C, $R_1$ = 7-$CH_3$, $R_2$ = $R_3$ = H, Y = (diméthyl-2,4 imidazolyl-1)-5, Z = pyridyl-4

Point de fusion : 286 °C

Exemple 155 **(Imidazolyl-1)-4 (pyridyl-4)-2 benzimidazole**

**Formule (I)** A = B = T = W = C, $R_1$ = $R_2$ = $R_3$ = H, Y = (imidazolyl-1)-4, Z = pyridyl-4

Point de fusion : 313 °C
Point de fusion du dichlorhydrate : 320-325 °C

Exemple 156 **Méthyl-6 (méthyl-4 imidazolyl-1)-5 (pyridyl-4)-2 benzimidazole**

**Formule (I)** A = B = T = W = C, $R_1$ = 6-$CH_3$, $R_2$ = $R_3$ = H, Y = (méthyl-4 imidazolyl-1)-5, Z = pyridyl-4

Point de fusion : 332 °C

Exemple 157 **Méthyl-6 (méthyl-2 imidazolyl-1)-5 (pyridyl-4)-2 benzimidazole**

**Formule (I)** A = B = T = W = C, $R_1$ = 6-$CH_3$, $R_2$ = $R_3$ = H, Y = (méthyl-2 imidazolyl-1)-5, Z = pyridyl-4

Point de fusion : 308 °C

Exemple 158 **(Diméthyl-2,4 imidazolyl-1)-5 méthyl-6 (pyridyl-4)-2 benzimidazole**

**Formule (I)** A = B = T = W = C, $R_1$ = 6-$CH_3$, $R_2$ = $R_3$ = H, Y = (diméthyl-2,4 imidazolyl-1)-5, Z = pyridyl-4

Point de fusion : 295 °C

Exemple 159 **(imidazolyl-1)-5 méthyl-6 [(imidazolyl-1)-4 phényl]-2 benzimidazole**

**Formule (I)** A = B = T = W = C, $R_1$ = 6-$CH_3$, $R_2$ = $R_3$ = H, Y = (imidazolyl-1)-5, Z = [(imidazolyl-1)-4 phényl]

Point de fusion : 300 °C

Exemple 160 **Chloro-6 (méthyl-2 imidazolyl-1)-5 (pyridyl-4)-2 benzimidazole**

**Formule (I)** A = B = T = W = C, $R_1$ = 6-Cl, $R_2$ = $R_3$ = H, Y = (méthyl-2 imidazolyl-1)-5, Z = pyridyl-4

Point de fusion : 324 °C

Exemple 161 **Chloro-6 (méthyl-4 imidazolyl-1)-5 (pyridyl-4)-2 benzimidazole**

**Formule (I)** A = B = T = W = C, $R_1$ = 6-Cl, $R_2$ = $R_3$ = H, Y = (méthyl-4 imidazolyl-1)-5, Z = pyridyl-4

Point de fusion : 285 °C

Exemple 162 **(imidazolyl-1)-5 méthyl-4 (pyridyl-4)-2 benzimidazole**

**Formule (I)** A = B = T = W = C, $R_1$ = 4-$CH_3$, $R_2$ = $R_3$ = H, Y = (imidazolyl-1)-5, Z = pyridyl-4

Point de fusion : 283 °C

Exemple 163 **(imidazolyl-1)-5 (pyridyl-4)-2 imidazo[4,5b] pyridine**

**Formule (I)** A = N, B = T = W = C, $R_1$ = $R_2$ = $R_3$ = H, Y = (imidazolyl-1)-2, Z = pyridyl-4

Point de fusion : 358-359 ° C

Exemple 164 **(imidazolyl-1)-5 (méthylthio-2 pyridyl-3)-2 benzimidazole**

**Formule (I)** A = B = T = W = C, $R_1$ = $R_2$ = $R_3$ = H, Y = (imidazolyl-1)-5, Z = (méthylthio-2 pyridyl-3)

Point de fusion : 217-218 ° C

Exemple 165 **(imidazolyl-1)-2 (pyridyl-4)-8 purine**

**Formule (I)** A = T = N, B = W = C, $R_1$ = $R_2$ = $R_3$ = H, Y = (imidazolyl-1)-5, Z = (pyridyl-4)

Point de fusion : 340 ° C (décomposition)

Exemple 166 **(imidazolyl-1)-5 (pyridyl-4)-2 trifluorométhyl-6 benzimidazole**

**Formule (I)** A = B = T = W = C, $R_1$ = 6-$CF_3$, $R_2$ = $R_3$ = H, Y = (imidazolyl-1)-5, Z = (pyridyl-4)

Point de fusion : 340 ° C

Exemple 167 **Chloro-6 (imidazolyl-1)-5 méthyl-7 (pyridyl-4)-2 benzimidazole**

**Formule (I)** A = B = T = W = C, $R_1$ = 6-Cl, $R_2$ = 7-$CH_3$, $R_3$ = H, Y = (imidazolyl-1)-5, Z = (pyridyl-4)

Point de fusion : 316 ° C

Exemple 168 **Diméthyl-6,7 (imidazolyl-1)-5 (pyridyl-4)-2 benzimidazole**

**Formule (I)** A = B = T = W = C, $R_1$ = 6-$CH_3$, $R_2$ = 7-$CH_3$, $R_3$ = H, Y = (imidazolyl-1)-5, Z = (pyridyl-4)

Point de fusion : 318 ° C.

Exemple 169 **[((Pyridyl-4)-2 benzimidazolyl-5)-1 méthyl-2 imidazolyl-4]-2 méthyl-2 dioxolane-1,3**

**Formule (I)** A = B = T = W = C, $R_1$ = $R_2$ = $R_3$ = H, Y = (methyl-2 dioxolane-1,3 yl-2)-4 méthyl-2 imidazolyl, Z = -(pyridyl-4)

Point de fusion : 255 ° C.

Exemple 170 **Ethyl-7 (imidazolyl-1)-5 (pyridyl-4)-2** benzimidazole

**Formule (I)** A = B = T = W = C, $R_1$ = 7-$CH_2$ $CH_3$, $R_2$ = $R_3$ = H, Y = (imidazolyl-1)-5, Z = (pyridyl-4)

Point de fusion : 280 ° C.

Exemple 171 **(Imidazolyl-1)-5 (chloro-6 pyridyl-3)-2 benzimidazole**

**Formule (I)** A = B = T = W = C, $R_1$ = $R_2R_3$ = H, Y = (imidazolyl-1)-5, Z = (chloro-6 pyridyl-3)

A une solution de 10,2 g de (imidazolyl-1)-5 nitro-2 aniline préparée à l'exemple 46 dans 100 ml de pyridine, introduire goutte à goutte 8,8 g de chlorure de l'acide chloro-6 nicotinique dans 50 ml de toluène. La réaction est exothermique. Agiter 3 h à température ambiante puis 3 h à 50° C. Concentrer sous vide. Reprendre à l'eau, essorer le solide obtenu, laver à l'eau puis à l'isopropanol pour avoir 13,7 g de chloro-6 N[(imidazolyl-1)-5 nitro-2 phényl] nicotinamide.

Point de fusion : 180° C.

L'hydrogénation catalytique de ce composé dans le méthoxy éthanol en présence de nickel de Raney selon l'exemple 70 est suivie d'un traitement dans 100 ml d'acide acétique au reflux pendant 3 h. Le composé obtenu est purifié partiellement sur gel de silice puis recristallisé dans le méthoxy-2 éthanol pour donner 4,8 g de (imidazolyl-1)-5 (chloro-6 pyridyl-3)-2 benzimidazole.

Point de fusion : > 330° C.

## TABLEAU

| Code | Formule | Exemple |
|---|---|---|
| 8057-12 | 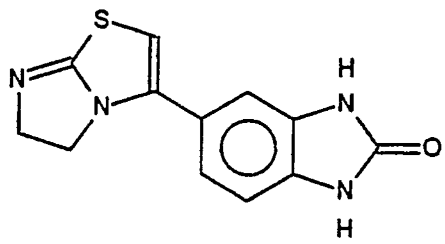 , 3 / 2 FUMARATE | 105 |
| 66-1 | 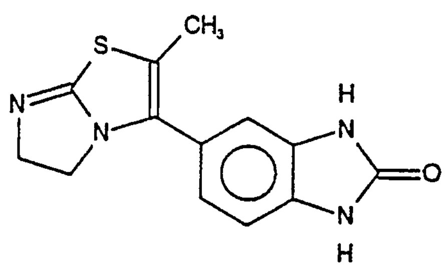 , HCL | 106 |
| 66-9 | 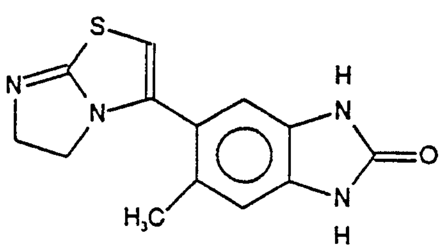 , HCL | 107 |
| 66-28 | 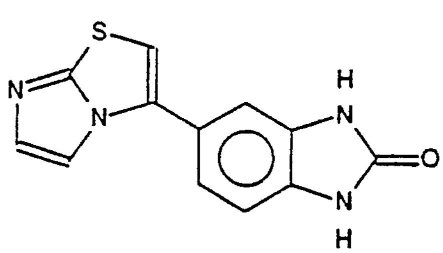 | 108 |

| Code | Formule | Exemple |
|---|---|---|
| 49-6 | 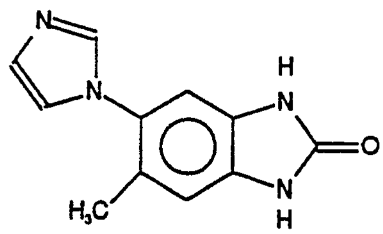 | 109 |
| 49-2 | 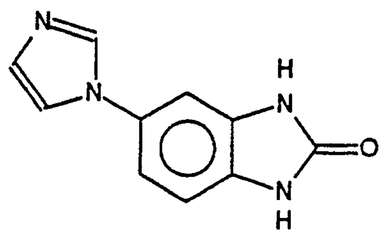 , HCL | 110 |
| 49-3 | 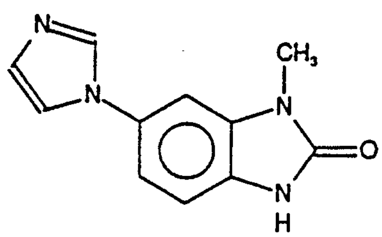 | 111 |
| 49-4 | 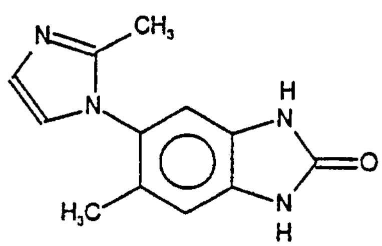 , HCL | 112 |

| Code | Formule | Exemple |
|---|---|---|
| 49-5 | 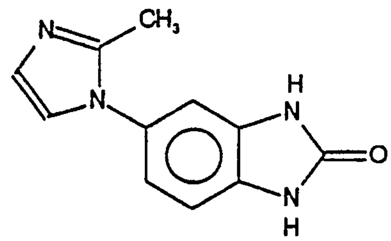 , HCL | 113 |
| 49-8 | 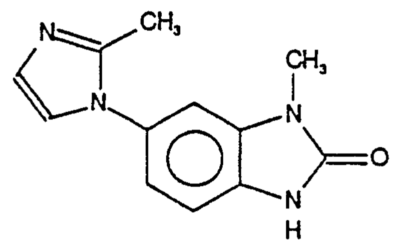 , HCL, $H_2O$ | 114 |
| 49-9 | 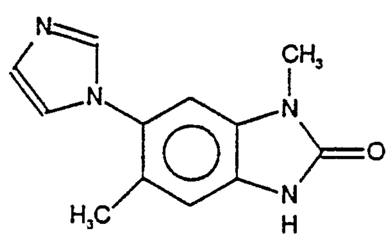 , HCL, $H_2O$ | 115 |
| 49-10 | 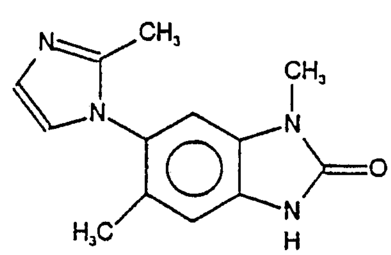 , HCL, $H_2O$ | 116 |

| Code | Formule | Exemple |
|---|---|---|
| 49-14 | 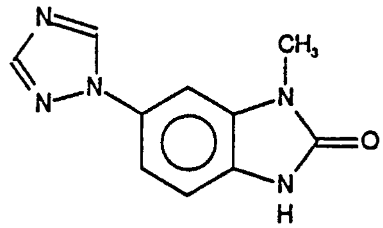 ,HCL | 117 |
| 49-18 | 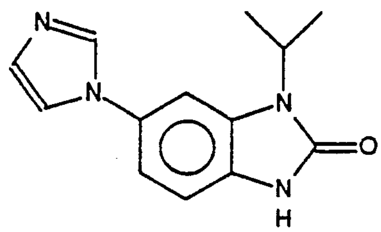 ,HCL | 118 |
| 49-19 | 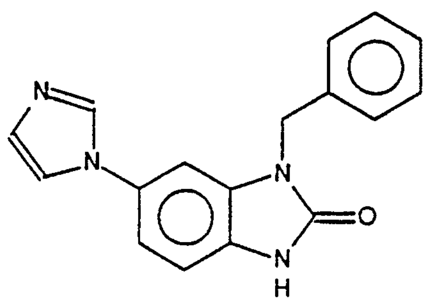 | 119 |
| 49-20 | 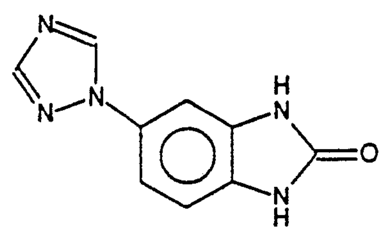 | 120 |

| Code | Formule | Exemple |
|---|---|---|
| 49-24 | 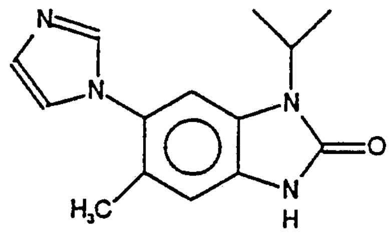 | 121 |
| 49-27 | 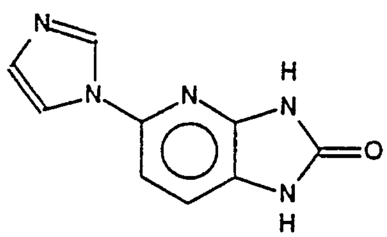 ,HCL | 122 |
| 49-7 | 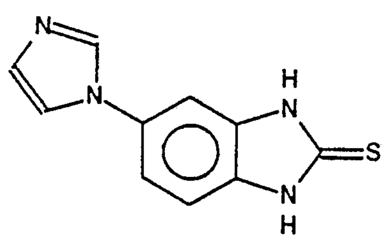 | 123 |
| 49-13 | 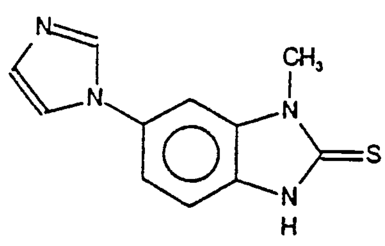 | 124 |

| Code | Formule | Exemple |
|---|---|---|
| 49-16 | 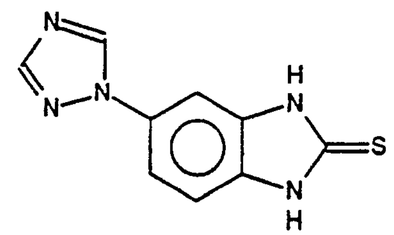 | 125 |
| 49-23 | 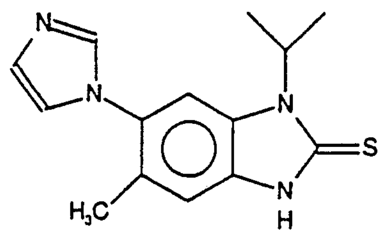 | 126 |
| 49-25 | 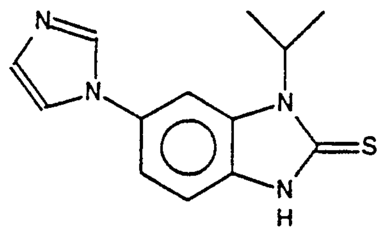 | 127 |
| 49-11 | 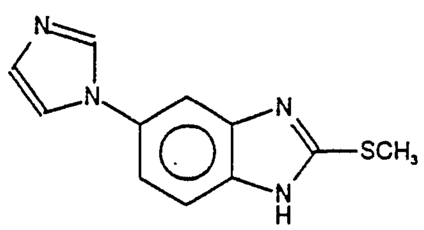 | 128 |

| Code | Formule | Exemple |
|---|---|---|
| 49-15 | 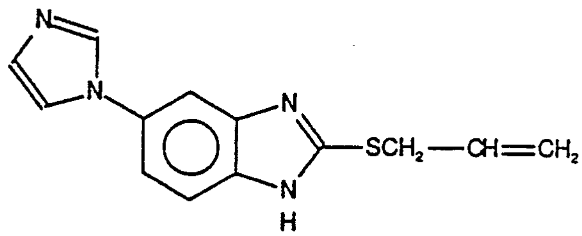 | 129 |
| 49-17 | 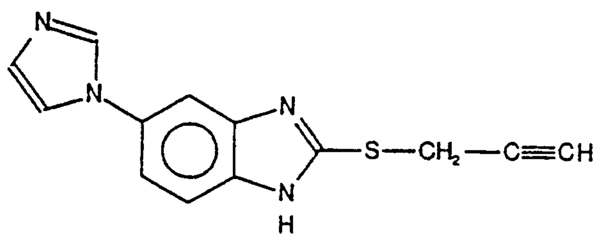 | 130 |
| 49-21 | 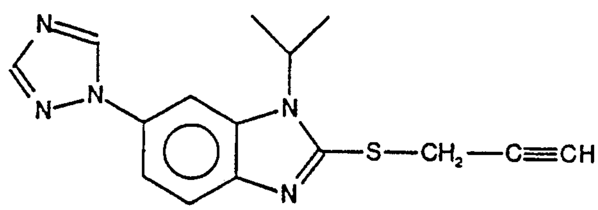 | 131 |
| 49-22 | 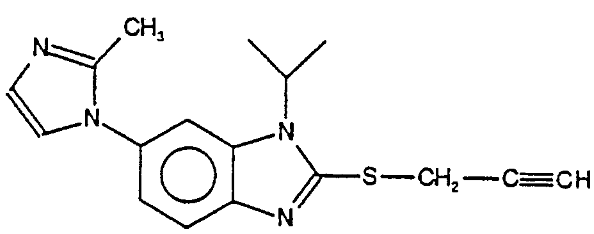 | 132 |

| Code | Formule | Exemple |
|---|---|---|
| 49-12 | 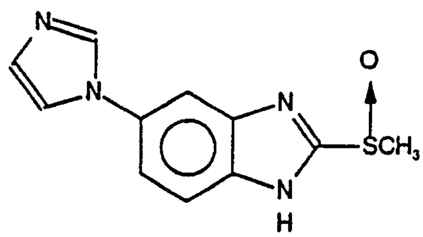 | 133 |
| 100-6 | 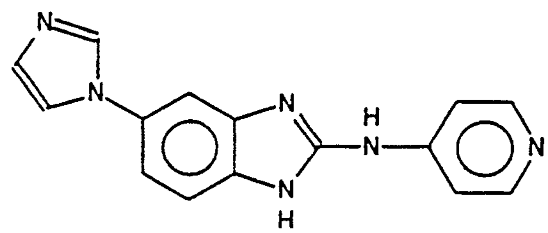 | 134 |
| 100-1 | 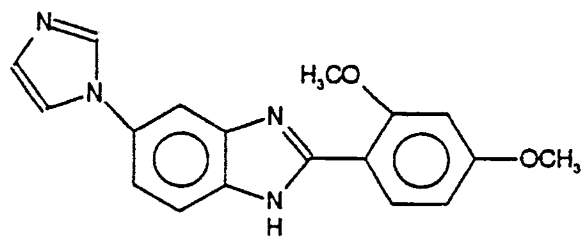 | 135 |
| 100-2 | 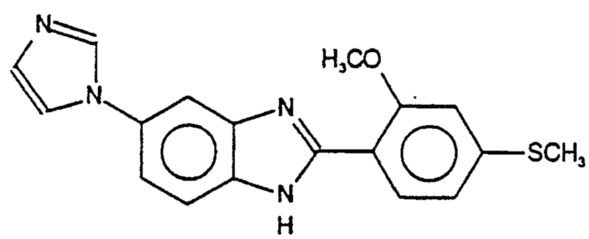 | 136 |

| Code | Formule | Exemple |
|---|---|---|
| 100-4 | 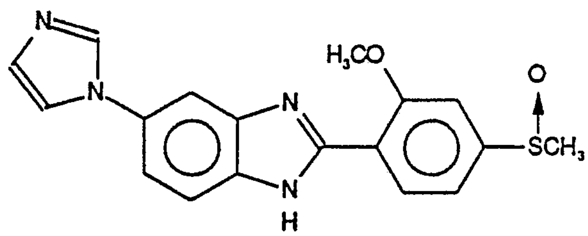 | 137 |
| 100-3 | 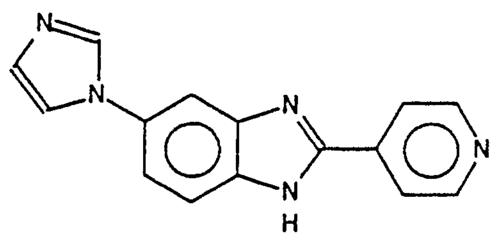 | 138 |
| 100-5 | 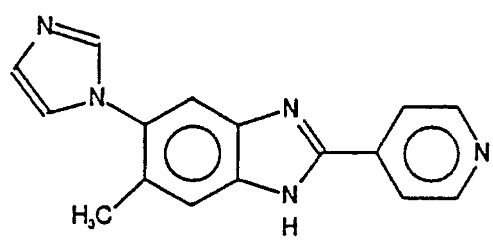 | 139 |
| 100-7 | 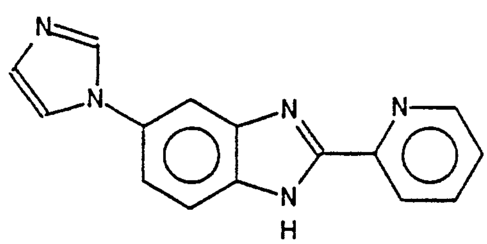 | 140 |

| Code | Formule | Exemple |
|---|---|---|
| 100-8 | 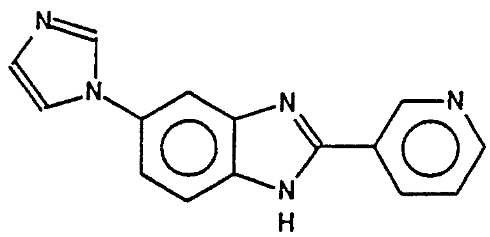 | 141 |
| 100-10 | 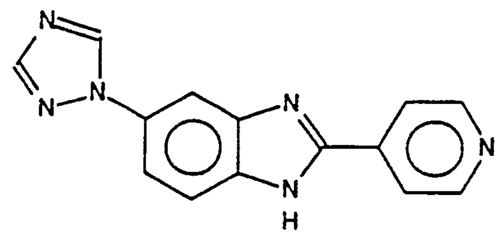 | 142 |
| 100-11 | 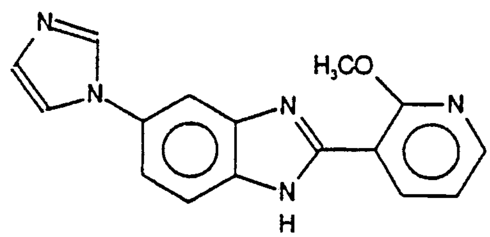 | 143 |
| 100-9 | 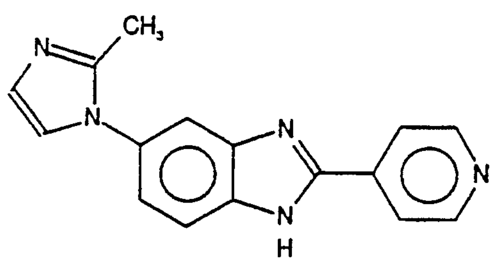 | 144 |

| Code | Formule | Exemple |
|---|---|---|
| 100-14 | 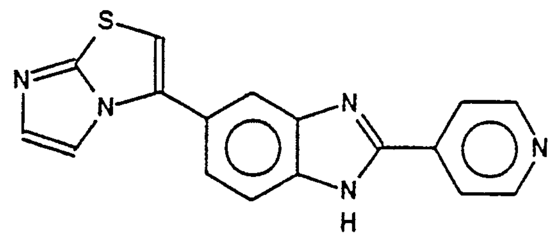 | 145 |
| 100-16 | 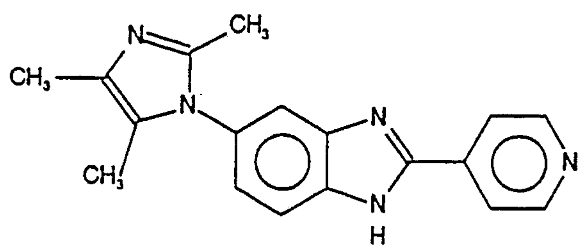 | 146 |
| 100-17 | 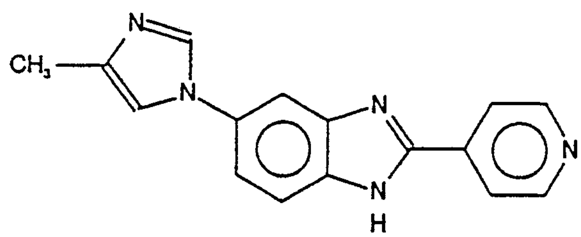 | 147 |
| 100-18 | 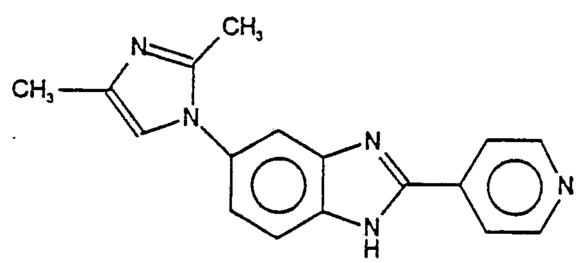 | 148 |

| Code | Formule | Exemple |
|---|---|---|
| 100-20 | 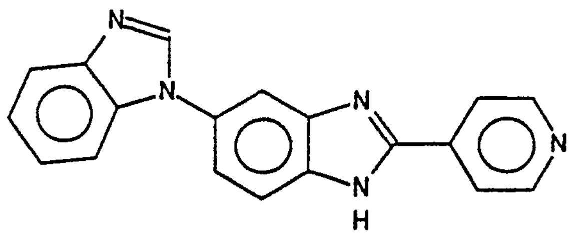 | 149 |
| 100-21 | 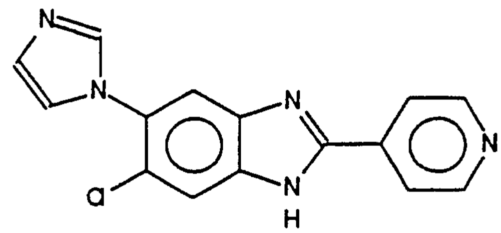 | 150 |
| 100-22 | 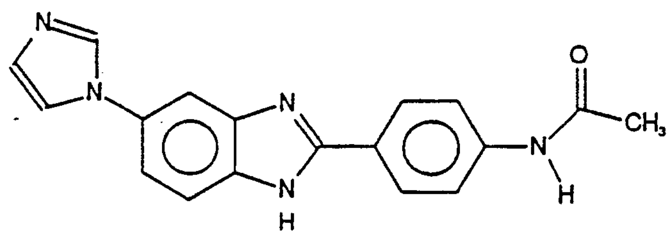 | 151 |
| 100-23 | 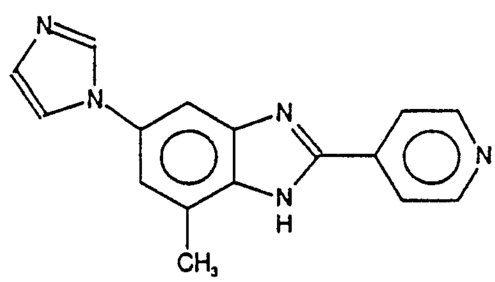 | 152 |

| Code | Formule | Exemple |
|---|---|---|
| 100-24 | 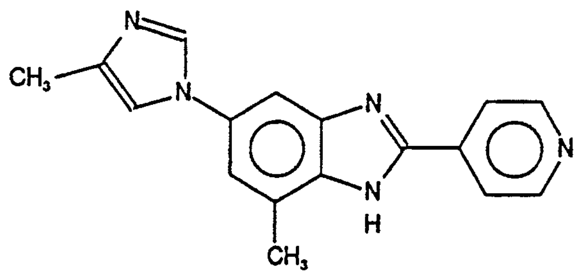 | 153 |
| 100-25 | 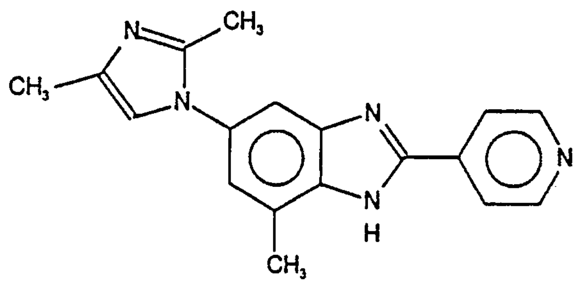 | 154 |
| 100-26 | 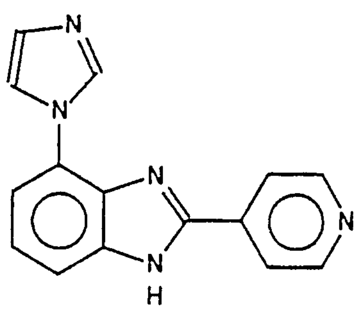 | 155 |
| 100-27 | 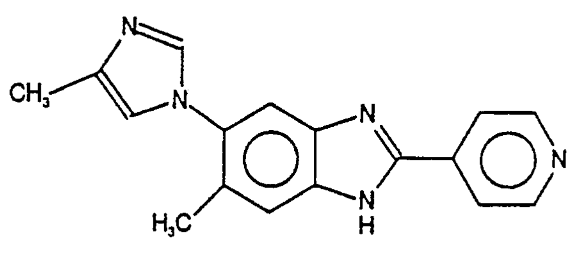 | 156 |

| Code | Formule | Exemple |
|---|---|---|
| 100-28 | 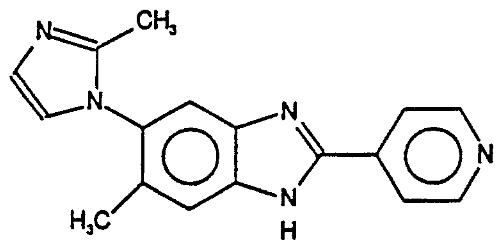 | 157 |
| 100-29 | 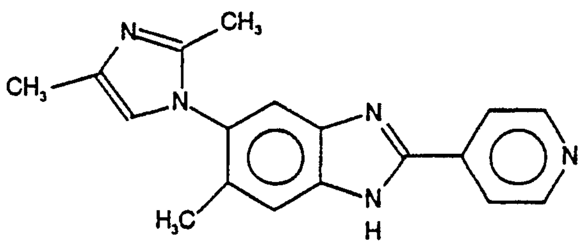 | 158 |
| 100-30 | 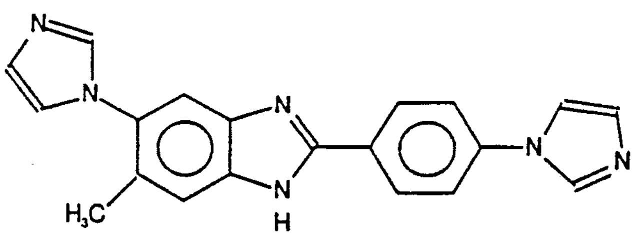 | 159 |
| 100-31 | 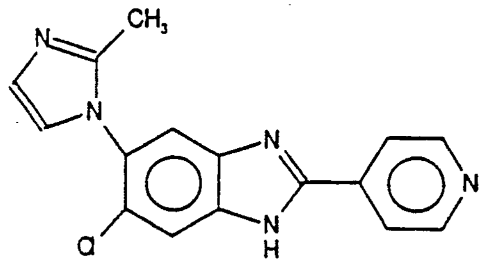 | 160 |

| Code | Formule | Exemple |
| --- | --- | --- |
| 100-32 | 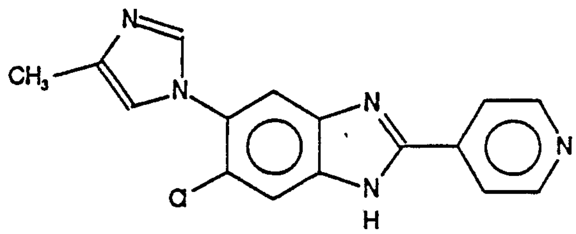 | 161 |
| 100-33 | 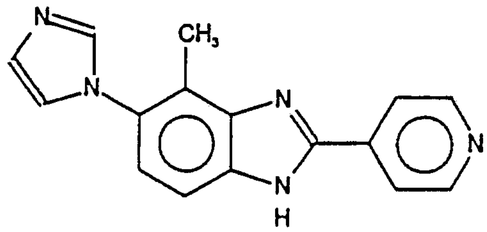 | 162 |
| 100-34 | 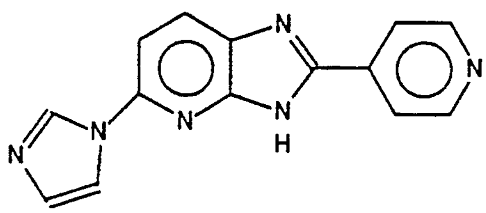 | 163 |
| 100-35 | 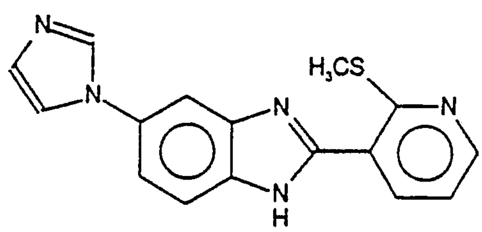 | 164 |

| Code | Formule | Exemple |
|---|---|---|
| 100-36 | 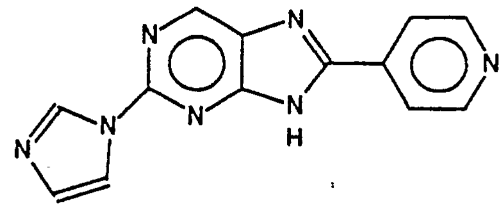 | 165 |
| 100-37 | 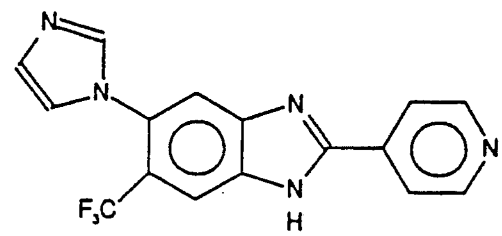 | 166 |
| 100-38 | 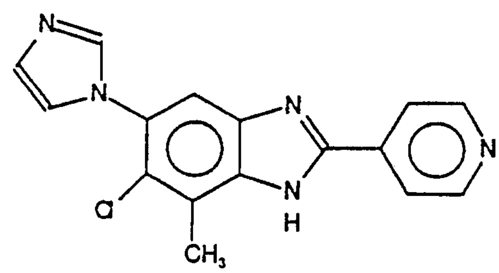 | 167 |
| 100-39 | 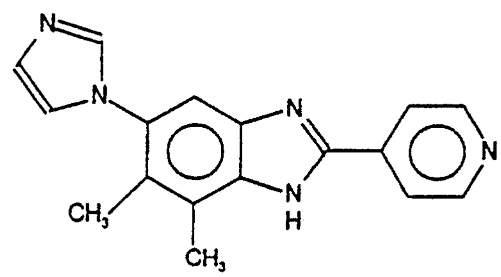 | 168 |

| Code | Formule | Exemple |
| --- | --- | --- |
| 100-40 | 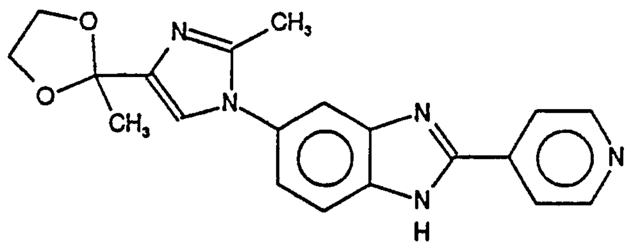 | 169 |
| 100-41 | 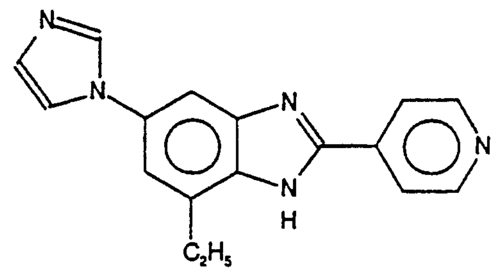 | 170 |
| 100-12 | 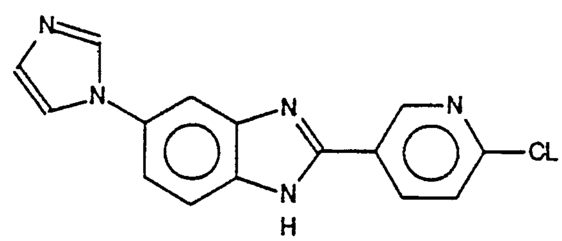 | 171 |

## PHARMACOLOGIE

Les produits des exemples revendiqués présentent des propriétés inotropes positives et anti ulcères. Les méthodes suivantes ont été utilisées pour mettre en évidence et quantifier ces activités.

**Activité inotrope positive**

Principe:

L'activité inotrope positive a été appréciée sur la préparation d'oreillette gauche isolée de cobaye stimulée à fréquence constante.

L'enregistrement de la force de contraction de l'oreillette est utilisé pour définir l'effet inotrope positif.

Mode opératoire:

Des cobayes mâles tricolores sont assommés et saignés. Le coeur est prélevé rapidement et rincé dans une solution de Krebs-Henseleit carbogénée. Les oreillettes sont séparées des ventricules et isolées l'une de l'autre. La partie basale de l'oreillette gauche est fixée sur le porte-organe entre deux électrodes et la partie libre est reliée à l'aide d'un fil à un capteur de tension isométrique. L'organe est plongé dans une cuve de 50 ml thermostatée à 32 °C et contenant une solution de Krebs-Henseleit. La préparation est soumise à une tension de 1 g, puis stimulée par des impulsions électriques rectangulaires de fréquence 3 Hz, de durée 1 ms et de voltage 20 à 30 % supérieur au voltage seuil (1 à 4 V). La force de contraction est mesurée à l'aide d'un capteur de tension relié à un enregistreur. La préparation est alors laissée au repos durant une période de 60 minutes pendant laquelle le milieu de survie est renouvelé toutes les 15 minutes et la tension réajustée si nécessaire.

Expression des résultats:

La force de contraction est mesurée après 5 minutes de contact avec la préparation.

A chaque fois que cela est possible, une concentration active 50 (M) et ses limites de confiance à $p < 0.05$ sont calculées. Elle exprime la concentration de produit induisant une augmentation de l'inotropisme égale à 50 % de l'effet inotrope maximum observé, exprimé par la tension maximale enregistrée.

| Produit de l'exemple | N° de code | CA50 - (M) et intervalle de confiance | Différence de tension maximum développée en mg (m±sem) |
|---|---|---|---|
| 81 | 100-3 | 8.8 E-5 (7.2 E-5 ; 1.1 E-4) | 558 ± 68 |
| 54 | 49-2 | 9.4 E-5 | 442 |
| 55 | 49-3 | 3.5 E-5 | 494 |
| 57 | 49-5 | 9.6 E-5 (7.4 E-5 ; 1.3 E-4) | 488 ± 54 |
| 53 | 49-6 | 4.5 E-5 (3.6 E-5 ; 5.5 E-5) | 682 ± 121 |
| 66 | 49-7 | 6.0 E-5 (4.8 E-5 ; 7.5 E-5) | 425 ± 110 |
| 58 | 49-8 | 4.9 E-5 | 283 ± 30 |
| 59 | 49-9 | 5.4 E-5 (4.6 E-5 ; 6.2 E-5) | 558 ± 61 |
| 71 | 49-11 | 6.0 E-5 (5.5 E-5 ; 7.3 E-5) | 604 ± 84 |
| 76 | 49-12 | 4.0 E-4 (3.3 E-4 ; 4.8 E-4) | 463 ± 155 |
| 67 | 49-13 | 2.7 E-5 | 275 ± 63 |
| 61 | 49-14 | 2.0 E-5 | 529 ± 90 |
| 72 | 49-15 | 1.8 E-5 (1.6 E-5 ; 2.2 E-5) | 496 ± 62 |
| 68 | 49-16 | 1.4 E-4 (1.2 E-4 ; 1.8 E-4) | 513 ± 61 |

| Produit de l'exemple | N° de code | CA50 - (M) et intervalle de confiance | Différence de tension maximum développée en mg (m ± sem) |
|---|---|---|---|
| 73 | 49-17 | 9.7 E-6 (7.7 E-6 ; 1.2 E-5) | 563 ± 55 |
| 62 | 49-18 | 1.1 E-5 (9.0 E-6 ; 1.4 E-5) | 608 ± 52 |
| 63 | 49-19 | 1.4 E-5 (1.0 E-5 ; 1.8 E-5) | 338 ± 38 |
| 64 | 49-20 | 3.7 E-4 (2.7 E-4 ; 5.1 E-4) | 364 ± 90 |
| 69 | 49-23 | 1.9 E-5 (1.6 E-5 ; 2.2 E-5) | 391 ± 46 |
| 49 | 8057-12 | 1.1 E-5 (6.0 E-5 ; 1.9 E-5) | 746 |
| 50 | 66-1 | 2.0 E-4 | 325 |
| 51 | 66-9 | 1.4 E-5 | 669 |
| 52 | 66-28 | 2.0 E-5 (1.2 E-5 ; 3.6 E-5) | 258 ± 36 |
| 134 | 100-6 | 6.5 E-5 (4.9 E-5 ; 8.4 E-5) | 533 ± 89 |
| 139 | 100-5 | 2.4 E-5 (1.7 E-5 ; 3.5 E-5) | 670 ± 98 |
| 141 | 100-8 | 8.2 E-5 (6.7 E-5 ; 1.01 E-4) | 625 ± 69 |
| 142 | 100-10 | 1.5 E-4 (8 E-5 ; 3 E-4) | 455 ± 102 |
| 143 | 100-11 | 1.4 E-4 (9 E-5 ; 2.10 E-4) | 496 ± 82 |

| Produit de l'exemple | N° de code | CA50 - (M) et intervalle de confiance | Différence de tension maximum développée en mg (m ± sem) |
|---|---|---|---|
| 144 | 100-9 | 4.3 E-5 (3.5 E-5 ; 5.3 E-5) | 708 ± 89 |
| 146 | 100-16 | 15 E-5 (8.10 E-6 ; 2.19 E-5) | 518 ± 180 |
| 148 | 100-18 | 2.6 E-5 (2.20 E-5 ; 2.80 E-5) | 337 ± 77 |
| 149 | 100-20 | 1.4 E-5 (1.1 E-5 ; 1.9 E-5) | 378 ± 68 |
| 152 | 100-23 | 1.3 E-5 (9 E-7 ; 1.8 E-5) | 443 ± 76 |
| 156 | 100-27 | 5.6 E-5 (3.27 E-5 ; 7.9 E-5) | 490 ± 87 |
| 157 | 100-28 | 5.8 E-5 (3.97 E-5 ; 7.63 E-5) | 585 ± 81 |
| 158 | 100-29 | 3.7 E-5 (2.8 E-5 ; 4.5 E-5) | 453 ± 52 |
| 160 | 100-31 | 3.9 E-5 (3.56 E-5 ; 4.24 E-5) | 620 ± 73 |

**Activité anti-ulcère**

Méthode

Des lots de 10 à 30 rats mâles de souche OFA (Iffa Credo), pesant 160-180 g, sont mis à la diète hydrique 24 h avant l'administration orale du produit étudié. Trente minutes plus tard, un agent ulcérigène médicamenteux ou un agent nécrosant (exemple : éthanol absolu) est administré par voie orale à des doses qui entraînent chez les animaux témoins une ulcération gastrique maximale. Les estomacs sont prélevés, selon le test, respectivement 6 et 1 h après le dernier traitement. Les lésions gastriques sont alors évaluées macroscopiquement (cotation et mesure de l'ampleur des ulcères).

Expression des résultats

Ils sont exprimés sous forme de dose active 50 déterminée graphiquement. Cette dose exprime la dose nécessaire pour réduire de 50 % les lésions ulcéreuses observées chez les animaux témoins.

| Résultats | | | |
|---|---|---|---|
| **Produit de l'exemple** | **N° de code** | **activité anti-ulcère vis à vis d'un agent nécrosant DA 50 Mg/Kg v.o.** | **activité anti-ulcère vis à vis d'un anti-inflammatoire non stéroïdien DA 50 Mg/Kg v.o.** |
| 123 | 49-7 | 11, 11 | - |
| 128 | 49-11 | 4,5 | 18,29 |
| 124 | 49-13 | 4,5 | - |
| 129 | 49-15 | 0,33 | - |
| 130 | 49-17 | 0,48 | 9,24 |

## TOXICOLOGIE

Des études préliminaires de toxicité ont pu montrer que les doses létales 50 déterminées après administration orale chez le rat étaient supérieures à 300 mg.kg-1, traduisant un index thérapeutique intéressant.

## CONCLUSION

En conclusion, les molécules décrites dans la présente demande possèdent des propriétés inotropes positives;anti-agrégantes plaquettaires et anti ulcères particulièrement intéressantes.

La stimulation de la fonction myocardique induite par ces dérivés ainsi que la protection induite vis-à-vis des lésions ulcérigènes peut être utilisée avec intérêt et bénéfice dans le traitement de l'insuffisance cardiaque et de l'ulcère gastroduodénal, par voie orale sous forme de comprimés ou de gélules dosés de 150 à 500 mg ou, par voie parentérale sous forme de préparations injectables dosées de 30 à 200 mg.

L'invention couvre également un procédé de préparation d'une composition pharmaceutique, caractérisé en ce qu'il comprend l'incorporation d'une quantité pharmaceutiquement efficace d'au moins un composé de formule (I) tel que précédemment défini, ou un de ses sels d'addition d'acide pharmacologiquement acceptable, dans un excipient, véhicule ou support pharmaceutiquement acceptable.

L'invention couvre également un procédé de traitement thérapeutique d'un mammifère incluant un être humain, caractérisé en ce qu'on administre à ce mammifère une quantité thérapeutiquement efficace d'au moins un composé de formule (I) tel que précédemment défini, ou un de ses sels d'addition d'acide pharmacologiquement acceptable, éventuellement incorporé dans un excipient, véhicule ou support pharmaceutiquement acceptable.

Selon une variante de réalisation, ce procédé de traitement thérapeutique consiste à un traitement des maladies cardiovasculaires.

Selon un autre mode de réalisation particulier, ce procédé de traitement concerne le traitement thérapeutique des ulcères gastroduodénaux.

**Revendications**

1. Dérivés de benzimidazoles et d'azabenzimidazoles caractérisés en ce qu'ils répondent à la formule :

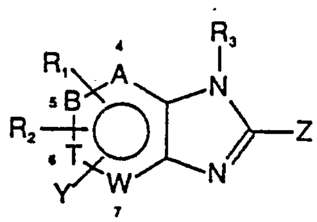

FORMULE (I)

et leurs formes tautomères dans laquelle :

-Y se trouve en position 4,5,6 ou 7 du noyau benzimidazole ou azabenzimidazole et représente un dérivé de l'imidazole, du benzimidazole, du triazole ou de l'imidazothiazole pouvant être ou non substitué par des groupements tels que :

Halogène, $COR_5$, $OR_5$ ,$SR_5$, $COOR_5$ ($R_5$ étant un atome d'hydrogène, un radical alkyle inférieur), un radical alkyle inférieur substitué ou non par des groupements halogène, $OR_5$, $SR_5$,$COOR_5$, $NHR_5$, $NHCOR_5$, $COR_5$, $R_5$ étant défini comme ci-dessus.

-Z peut représenter un cycle phényle ou pyridyle lié au benzimidazole ou à l'azabenzimidazole directement ou indirectement par l'intermédiaire d'un atome d'azote non substitué ou substitué par un radical alkyle inférieur en particulier l'aniline ou l'aminopyridine. Le cycle phényle ou pyridyle peut être ou non substitué notamment par un ou plusieurs radicaux alkyles inférieurs, un ou plusieurs atomes d'halogènes, un ou plusieurs groupements $OR_5$, $SR_5$, $SOR_5$, $NHCOR_5$, $NHR_5$ ($R_5$ étant défini comme ci-dessus), un hétérocycle de 5 à 10 atomes comportant 1 à 3 hétéroéléments choisis parmi l'azote, l'oxygène ou le soufre,

Z peut encore représenter un groupement OH, SH, $SR_6$ ou $SOR_6$, $R_6$ étant un alkyle inférieur, un alkényle en $C_2$-$C_8$, en particulier un allyle ou un alkynyle en $C_2$-$C_8$, en particulier un propargyle.

-$R_1$ et $R_2$ représentent simultanément ou non l'atome d'hydrogène, l'atome d'halogène ou un groupement $CF_3$, $NO_2$, $NHR_4$, $NHCOR_4$, $OR_4$,$SR_4$ ($R_4$ représentant un atome d'hydrogène ou un radical alkyle inférieur) ou un alkyle inférieur et peuvent se trouver en position 4,5,6,7 du benzimidazole ou de l'azabenzimidazole.

-$R_3$ représente l'atome d'hydrogène et peut représenter un radical alkyle inférieur ou un groupement benzyle lorsque Z représente un groupement OH, SH, $SR_6$ ou $SOR_6$.

-A, B, T, W peuvent représenter un atome de carbone ou un hétéro élément comme l'azote.

ainsi que leurs sels d'addition d'acides avantageusement pharmacologiquement acceptables.

2. Dérivés selon la revendication 1, caractérisés en ce que Y est l'imidazole.

3. Dérivés selon la revendication 1 ou 2, caractérisés en ce que Z est le radical pyridyle, en particulier pyridyl-4.

4. Dérivés selon la revendication 1 ou 2, caractérisés en ce que Z est l'aminopyridine et en particulier l'amino-4 pyridine.

5. Dérivés selon la revendication 1 ou 2, caractérisés en ce que Z est le groupement OH.

6. Dérivés selon la revendication 1 ou 2, caractérisés en ce que Z est le groupement SH.

7. Dérivés selon l'une des revendications 1 à 6, caractérisés en ce que $R_1$ est l'hydrogène.

8. Dérivés selon l'une quelconque des revendications 1 à 6, caractérisés en ce que $R_1$ est un radical alkyle inférieur en $C_1$-$C_6$, en particulier le méthyle, de préférence en position 6 ou 7.

9. Dérivés selon l'une des revendications 1 à 6, caractérisés en ce que $R_1$ est l'atome de chlore.

10. Dérivés selon l'une des revendications 1 à 6, caractérisés en ce que $R_1$ est le groupement trifluorométhyle.

11. Dérivés selon l'une des revendications 1 à 10, caractérisés en ce que $R_2$ est l'atome d'hydrogène.

12. Dérivés selon l'une des revendications 1 à 10, caractérisés en ce que $R_2$ est le radical méthyle.

13. Dérivés selon l'une des revendications 1 à 12, caractérisés en ce que $R_3$ est le radical isopropyle lorsque Z est le groupement OH ou SH.

14. Dérivés selon l'une des revendications 1 à 13, caractérisés en ce que W est l'atome d'azote.

15. Dérivés selon l'une des revendications 1 à 14, caractérisés en ce que B et W sont des atomes d'azote.

16. Dérivés selon les revendications 1, 2, 3, 8, 11 et 13 à 15, caractérisés en ce que $R_1$ est un radical alkyle inférieur, en particulier le méthyle, en position 7, $R_2$ est l'atome d'hydrogène, Y est un radical imidazolyle et Z est un radical pyridyl-4.

17. Dérivés selon l'une quelconque des revendications 1 à 16 caractérisés en ce qu'ils sont choisis parmi les produits de formule :

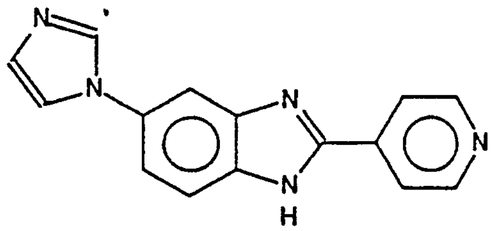

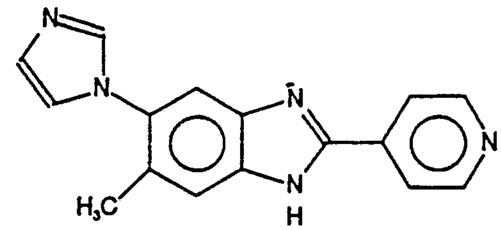

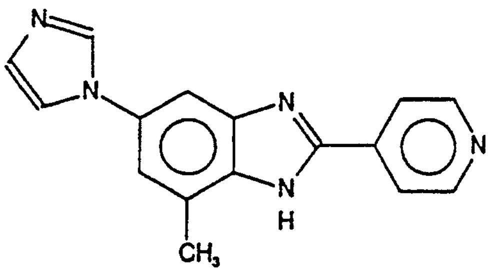

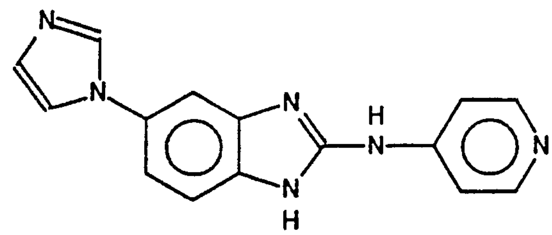

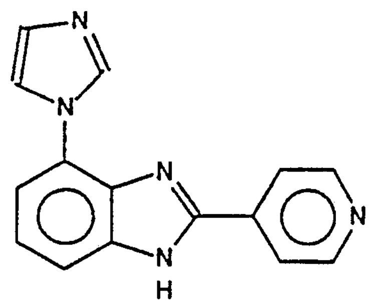

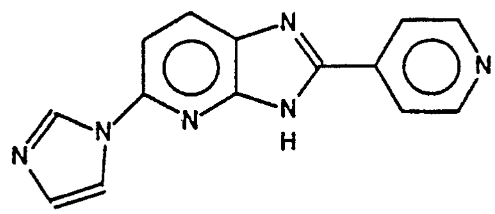

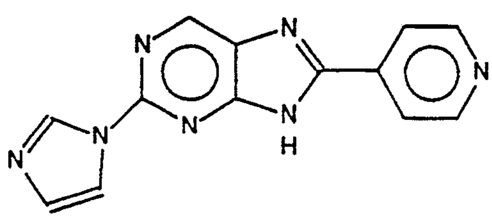

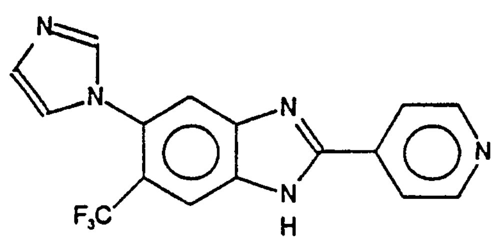

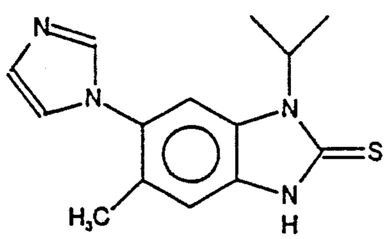

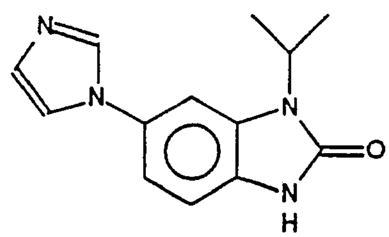

, HCL

18. Nouveau dérivé de benzimidazole caractérisé en ce qu'il présente la formule chimique développée suivante :

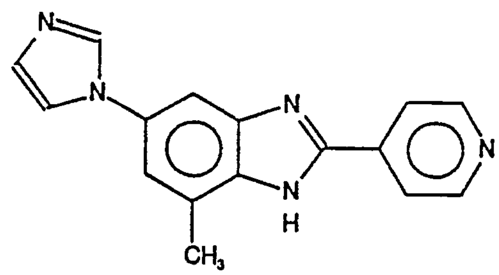

19. Procédés de préparation des composés de formule (I) selon l'une quelconque des revendications 1 à 18 caractérisés en ce qu'ils sont préparés par réaction de l'urée, de la thiourée, du sulfure de carbone, d'un aldéhyde benzénique ou pyridinique suivi d'une oxydation, d'un chlorure d'acide benzénique ou pyridinique, d'un acide benzoïque ou pyridinique, d'un nitrile benzénique ou pyridinique, d'un dithiocarbamate benzénique ou pyridinique, d'un isothiocyanate benzénique ou pyridinique ou d'un imidodithiocarbonate benzénique ou pyridinique sur des composés diaminés de formule (II) :

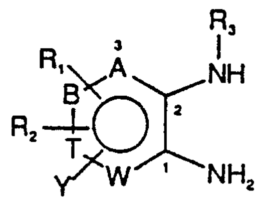

FORMULE (II)

dans laquelle
$R_1$ et $R_2$ représentent simultanément ou non l'atome d'hydrogène l'atome d'halogène ou un groupement $CF_3$, $NO_2$, $NHR_4$, $NHCOR_4$, $OR_4$,$SR_4$ ($R_4$ représentant un atome d'hydrogène ou un radical alkyle inférieur) ou un alkyle inférieur.
$R_3$ représente l'atome d'hydrogène, un alkyle inférieur ou un groupement benzyle.
Y représente un dérivé de l'imidazole, du benzimidazole, du triazole ou de l'imidazothiazole pouvant être ou non substitué par des groupements :
Halogène, $COR_5$, $OR_5$, $SR_5$, $COOR_5$ ($R_5$ étant un atome d'hydrogène ou un radical alkyle inférieur), un radical alkyle inférieur substitué ou non par des groupements halogènes, $OR_5$, $SR_5$, $COOR_5$, $NHR_5$, $NHCOR_5$, $COR_5$, $R_5$ étant défini comme ci-dessus.

20. Intermédiaires de synthèse de formule (II) tels que définis à la revendication 19 utiles pour la préparation des composés (I) correspondants tels que définis à l'une quelconque des revendications 1 à 18.

21. Composition pharmaceutique, caractérisée en ce qu'elle comprend une quantité pharmaceutiquement efficace d'au moins un composé de formule (I) tels que définis à l'une quelconque des revendications 1 à 18 ou un de ses sels d'addition d'acide pharmacologiquement acceptable, éventuellement incorporé dans un excipient, véhicule ou support pharmaceutiquement acceptable.

22. Composition pharmaceutique à activité cardiovasculaire, caractérisée en ce qu'elle comprend une quantité pharmaceutiquement efficace d'au moins un composé de formule (I) tel que défini dans l'une quelconque des revendications 1 à 18, ou un de ses sels d'addition d'acide pharmacologiquement acceptable, éventuellement incorporé dans un excipient, véhicule ou support pharmaceutiquement acceptable.

23. Composition pharmaceutique à activité anti-secrétoire et/ou anti-ulcère, caractérisée en ce qu'elle comprend une quantité pharmaceutiquement efficace d'au moins un composé de formule (I) tel que défini à l'une quelconque des revendications 1 à 18, éventuellement incorporé dans un excipient, véhicule ou support pharmaceutiquement acceptable.

24. Procédé de préparation d'une composition pharmaceutique, caractérisé en ce qu'on incorpore une quantité thérapeutiquement efficace d'au moins un composé de formule (I) tel que précédemment défini, à l'une des revendications 1 à 18, ainsi qu'éventuellement ses sels d'addition d'acides pharmacologiquement acceptables, dans un excipient, véhicule ou support pharmaceutiquement acceptable.

25. Procédé de préparation selon la revendication 24 caractérisé en ce qu'on prépare une composition

pharmaceutique à activité cardiovasculaire, cardiotonique, vasodilatatrice, anti-hypertensive et anti-agrégante plaquettaire ou anti-sécrétoire ou anti-ulcère.

Revendications pour l'Etat contractant suivant: GR

1. Dérivés de benzimidazoles et d'azabenzimidazoles caractérisés en ce qu'ils répondent à la formule :

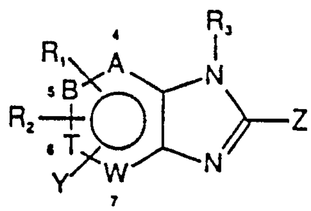

FORMULE (I)

et leurs formes tautomères dans laquelle :
-Y se trouve en position 4,5,6 ou 7 du noyau benzimidazole ou azabenzimidazole et représente un dérivé de l'imidazole, du benzimidazole, du triazole ou de l'imidazothiazole pouvant être ou non substitué par des groupements tels que :
Halogène, $COR_5$, $OR_5$ ,$SR_5$, $COOR_5$ ($R_5$ étant un atome d'hydrogène, un radical alkyle inférieur), un radical alkyle inférieur substitué ou non par des groupements halogène, $OR_5$, $SR_5$,$COOR_5$, $NHR_5$, $NHCOR_5$, $COR_5$, $R_5$ étant défini comme ci-dessus.
-Z peut représenter un cycle phényle ou pyridyle lié au benzimidazole ou à l'azabenzimidazole directement ou indirectement par l'intermédiaire d'un atome d'azote non substitué ou substitué par un radical alkyle inférieur en particulier l'aniline ou l'aminopyridine. Le cycle phényle ou pyridyle peut être ou non substitué notamment par un ou plusieurs radicaux alkyles inférieurs, un ou plusieurs atomes d'halogènes, un ou plusieurs groupements $OR_5$, $SR_5$, $SOR_5$, $NHCOR_5$, $NHR_5$ ($R_5$ étant défini comme ci-dessus), un hétérocycle de 5 à 10 atomes comportant 1 à 3 hétéroéléments choisis parmi l'azote, l'oxygène ou le soufre,
Z peut encore représenter un groupement OH, SH, $SR_6$ ou $SOR_6$, $R_6$ étant un alkyle inférieur, un alkényle en $C_2$-$C_8$, en particulier un allyle ou un alkynyle en $C_2$-$C_8$, en particulier un propargyle.
-$R_1$ et $R_2$ représentent simultanément ou non l'atome d'hydrogène, l'atome d'halogène ou un groupement $CF_3$, $NO_2$, $NHR_4$, $NHCOR_4$, $OR_4$,$SR_4$ ($R_4$ représentant un atome d'hydrogène ou un radical alkyle inférieur) ou un alkyle inférieur et peuvent se trouver en position 4,5,6,7 du benzimidazole ou de l'azabenzimidazole.
-$R_3$ représente l'atome d'hydrogène et peut représenter un radical alkyle inférieur ou un groupement benzyle lorsque Z représente un groupement OH, SH, $SR_6$ ou $SOR_6$.
-A, B, T, W peuvent représenter un atome de carbone ou un hétéro élément comme l'azote.
ainsi que leurs sels d'addition d'acides avantageusement pharmacologiquement acceptables.

2. Dérivés selon la revendication 1, caractérisés en ce que Y est l'imidazole.

3. Dérivés selon la revendication 1 ou 2, caractérisés en ce que Z est le radical pyridyle, en particulier pyridyl-4.

4. Dérivés selon la revendication 1 ou 2, caractérisés en ce que Z est l'aminopyridine et en particulier l'amino-4 pyridine.

5. Dérivés selon la revendication 1 ou 2, caractérisés en ce que Z est le groupement OH.

6. Dérivés selon la revendication 1 ou 2, caractérisés en ce que Z est le groupement SH.

7. Dérivés selon l'une des revendications 1 à 6, caractérisés en ce que $R_1$ est l'hydrogène.

8. Dérivés selon l'une quelconque des revendications 1 à 6, caractérisés en ce que $R_1$ est un radical alkyle inférieur en $C_1$-$C_6$, en particulier le méthyle, de préférence en position 6 ou 7.

9. Dérivés selon l'une des revendications 1 à 6, caractérisés en ce que $R_1$ est l'atome de chlore.

10. Dérivés selon l'une des revendications 1 à 6, caractérisés en ce que $R_1$ est le groupement trifluorométhyle.

11. Dérivés selon l'une des revendications 1 à 10, caractérisés en ce que $R_2$ est l'atome d'hydrogène.

12. Dérivés selon l'une des revendications 1 à 10, caractérisés en ce que $R_2$ est le radical méthyle.

13. Dérivés selon l'une des revendications 1 à 12, caractérisés en ce que $R_3$ est le radical isopropyle lorsque Z est le groupement OH ou SH.

14. Dérivés selon l'une des revendications 1 à 13, caractérisés en ce que W est l'atome d'azote.

15. Dérivés selon l'une des revendications 1 à 14, caractérisés en ce que B et W sont des atomes

d'azote.

16. Dérivés selon les revendications 1, 2, 3, 8, 11 et 13 à 15, caractérisés en ce que $R_1$ est un radical alkyle inférieur, en particulier le méthyle, en position 7, $R_2$ est l'atome d'hydrogène, Y est un radical imidazolyle et Z est un radical pyridyl-4.

17. Dérivés selon l'une quelconque des revendications 1 à 16 caractérisés en ce qu'ils sont choisis parmi les produits de formule :

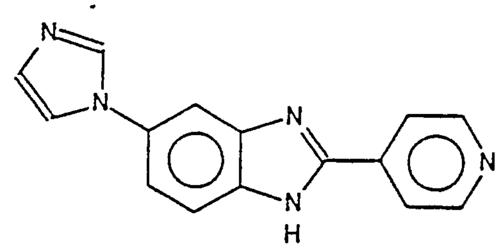

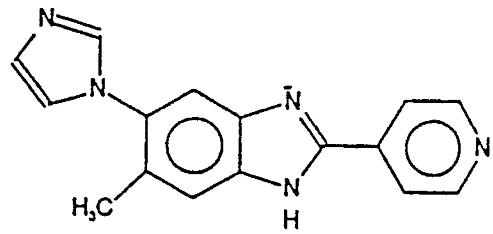

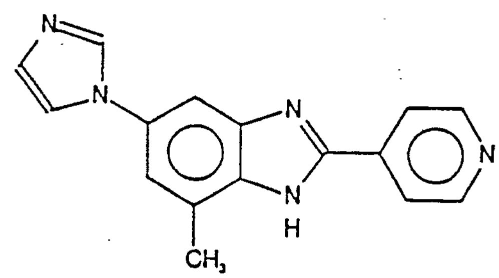

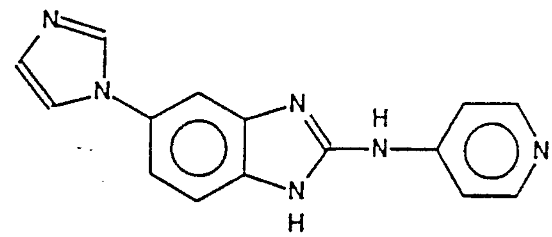

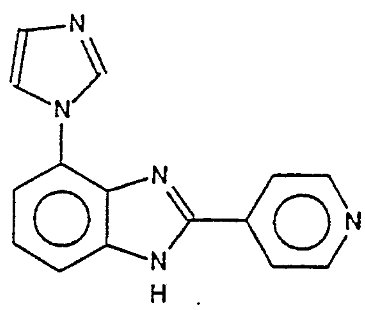

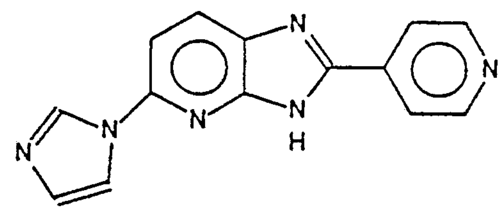

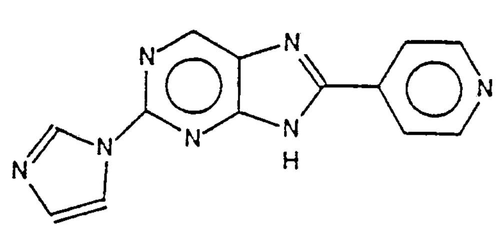

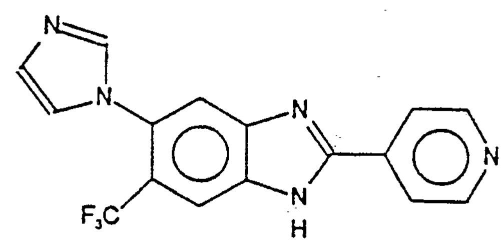

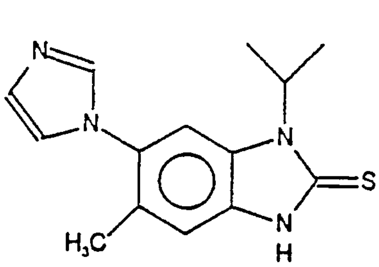

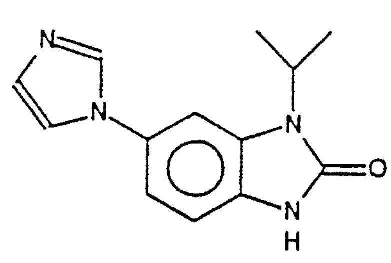

.HCL

18. Nouveau dérivé de benzimidazole caractérisé en ce qu'il présente la formule chimique développée suivante :

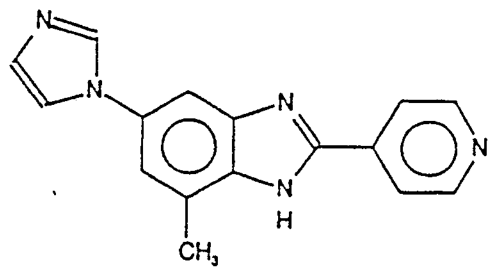

19. Procédés de préparation des composés de formule (I) selon l'une quelconque des revendications 1 à 18 caractérisés en ce qu'ils sont préparés par réaction de l'urée, de la thiourée, du sulfure de carbone, d'un aldéhyde benzénique ou pyridinique suivi d'une oxydation, d'un chlorure d'acide benzénique ou pyridinique, d'un acide benzoïque ou pyridinique, d'un nitrile benzénique ou pyridinique, d'un dithiocarbamate benzénique ou pyridinique, d'un isothiocyanate benzénique ou pyridinique ou d'un imidodithiocarbonate benzénique ou pyridinique sur des composés diaminés de formule (II) :

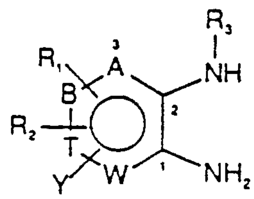

FORMULE (II)

dans laquelle

$R_1$ et $R_2$ représentent simultanément ou non l'atome d'hydrogène l'atome d'halogène ou un groupement $CF_3$, $NO_2$, $NHR_4$, $NHCOR_4$, $OR_4$,$SR_4$ ($R_4$ représentant un atome d'hydrogène ou un radical alkyle inférieur) ou un alkyle inférieur.

$R_3$ représente l'atome d'hydrogène, un alkyle inférieur ou un groupement benzyle.

Y représente un dérivé de l'imidazole,du benzimidazole,du triazole ou de l'imidazothiazole pouvant être ou non substitué par des groupements :

Halogène, $COR_5$, $OR_5$, $SR_5$, $COOR_5$ ($R_5$ étant un atome d'hydrogène ou un radical alkyle inférieur), un radical alkyle inférieur substitué ou non par des groupements halogènes, $OR_5$, $SR_5$, $COOR_5$, $NHR_5$, $NHCOR_5$, $COR_5$, $R_5$ étant défini comme ci-dessus.

20. Intermédiaires de synthèse de formule (II) tels que définis à la revendication 19 utiles pour la préparation des composés (I) correspondants tels que définis à l'une quelconque des revendications 1 à 18.

21. Procédé de préparation d'une composition pharmaceutique, caractérisé en ce qu'on incorpore une quantité thérapeutiquement efficace d'au moins un composé de formule (I) tel que précédemment défini, à l'une des revendications 1 à 18, ainsi qu'éventuellement ses sels d'addition d'acides pharmacologiquement acceptables, dans un excipient, véhicule ou support pharmaceutiquement acceptable.

22 . Procédé de préparation selon la revendication 21 caractérisé en ce qu'on prépare une composition pharmaceutique à activité cardiovasculaire, cardiotonique, vasodilatatrice, anti-hypertensive et anti-agrégante plaquettaire ou anti-sécrétoire ou anti-ulcère.

Revendications pour l'Etat contractant suivant: ES .

1. Procédé de préparation de dérivés de benzimidazoles et d'azabenzimidazoles caractérisé en ce qu'on prépare les dérivés répondant à la formule :

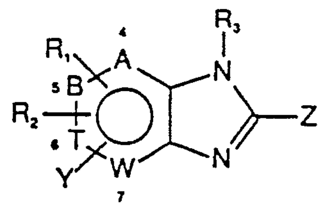

FORMULE (I)

et leurs formes tautomères dans laquelle :
-Y se trouve en position 4,5,6 ou 7 du noyau benzimidazole ou azabenzimidazole et représente un dérivé de l'imidazole, du benzimidazole, du triazole ou de l'imidazothiazole pouvant être ou non substitué par des groupements tels que :
Halogène, $COR_5$, $OR_5$ , $SR_5$, $COOR_5$ ($R_5$ étant un atome d'hydrogène, un radical alkyle inférieur), un radical alkyie inférieur substitué ou non par des groupements halogène, $OR_5$, $SR_5$,$COOR_5$, $NHR_5$, $NHCOR_5$, $COR_5$, $R_5$ étant défini comme ci-dessus.
-Z peut représenter un cycle phényle ou pyridyle lié au benzimidazole ou à l'azabenzimidazole directement ou indirectement par l'intermédiaire d'un atome d'azote non substitué ou substitué par un radical alkyle inférieur en particulier l'aniline ou l'aminopyridine. Le cycle phényle ou pyridyle peut être ou non substitué notamment par un ou plusieurs radicaux alkyles inférieurs, un ou plusieurs atomes d'halogènes, un ou plusieurs groupements $OR_5$, $SR_5$, $SOR_5$, $NHCOR_5$, $NHR_5$ ($R_5$ étant défini comme ci-dessus), un hétérocycle de 5 à 10 atomes comportant 1 à 3 hétéroéléments choisis parmi l'azote, l'oxygène ou le soufre,
Z peut encore représenter un groupement OH, SH, $SR_6$ ou $SOR_6$, $R_6$ étant un alkyle inférieur, un alkényle en $C_2$-$C_8$, en particulier un allyle ou un alkynyle en $C_2$-$C_8$, en particulier un propargyle.
-$R_1$ et $R_2$ représentent simultanément ou non l'atome d'hydrogène, l'atome d'halogène ou un groupement $CF_3$, $NO_2$, $NHR_4$, $NHCOR_4$, $OR_4$,$SR_4$ ($R_4$ représentant un atome d'hydrogène ou un radical alkyle inférieur) ou un alkyle inférieur et peuvent se trouver en position 4,5,6,7 du benzimidazole ou de l'azabenzimidazole.
-$R_3$ représente l'atome d'hydrogène et peut représenter un radical alkyle inférieur ou un groupement benzyle lorsque Z représente un groupement OH, SH, $SR_6$ ou $SOR_6$.
-A, B, T, W peuvent représenter un atome de carbone ou un hétéro élément comme l'azote.
ainsi que leurs sels d'addition d'acides avantageusement pharmacologiquement acceptables.

2. Procédé selon la revendication 1, caractérisé en ce qu'on prépare les composés dans lesquels Y est l'imidazole.

3. Procédé selon la revendication 1 ou 2, caractérisé en ce qu'on prépare les composés dans lesquels Z est le radical pyridyle, en particvulier pyridyl-4.

4. Procédé selon la revendication 1 ou 2, caractérisé en ce qu'on prépare les composés dans lesquels Z est l'aminopyridine et en particulier l'amino-4 pyridine.

5. Procédé selon la revendication 1 ou 2, caractérisé en ce qu'on prépare les composés dans lesquels Z est le groupement OH.

6. Procédé selon la revendication 1 ou 2, caractérisé en ce qu'on prépare les composés dans lesquels Z est le groupement SH.

7. Procédé selon l'une des revendications 1 à 6, caractérisé en ce qu'on prépare les composés dans lesquels $R_1$ est l'hydrogène.

8. Procédé selon l'une quelconque des revendications 1 à 6, caractérisé en ce qu'on prépare les composés dans lesquels $R_1$ est un radical alkyle inférieure en $C_1$-$C_6$, en particulier le méthyle, de préférence en position 6 ou 7.

9. Procédé selon l'une des revendications 1 à 6, caractérisé en ce qu'on prépare les composés dans lesquels $R_1$ est l'atome de chlore.

10. Procédé selon l'une des revendications 1 à 6, caractérisé en ce qu'on prépare les composés dans lesquels $R_1$ est le groupement trifluorométhyle.

11. Procédé selon l'une des revendications 1 à 10, caractérisé en ce qu'on prépare les composés dans lesquels $R_2$ est l'atome d'hydrogène.

12. Procédé selon l'une des revendications 1 à 10, caractérisé en ce qu'on prépare les composés dans lesquels $R_2$ est le radical méthyle.

13. Procédé selon l'une des revendications 1 à 12, caractérisé en ce qu'on prépare les composés dans lesquels $R_3$ est le radical isopropyle lorsque Z est le groupement OH ou SH.

14. Procédé selon l'une des revendications 1 à 13, caractérisé en ce qu'on prépare les composés dans

lesquels W est l'atome d'azote.

15. Procédé selon l'une des revendications 1 à 14, caractérisé en ce qu'on prépare les composés dans lesquels B et W sont des atomes d'azote.

16. Procédé selon l'une des revendications 1, 2, 3, 8, 11 et 13 à 15, caractérisé en ce qu'on prépare les composés dans lesquels $R_1$ est un radical alkyle inférieur, en particulier le méthyle, en position 7, $R_2$ est l'atome d'hydrogène, Y est un radical imidazolye et Z est un radical pyridyl-4.

17. Procédé selon l'une quelconque des revendications 1 à 16, caractérisé en ce qu'on prépare les produits de formule :

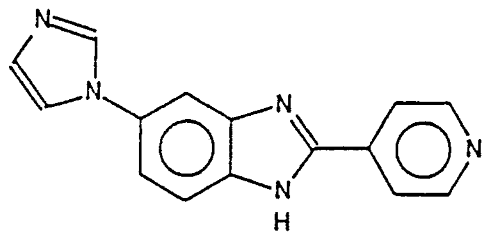
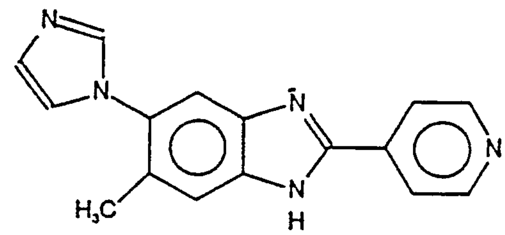
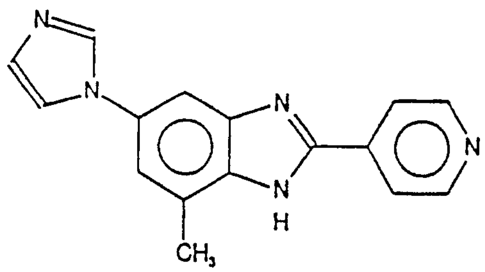
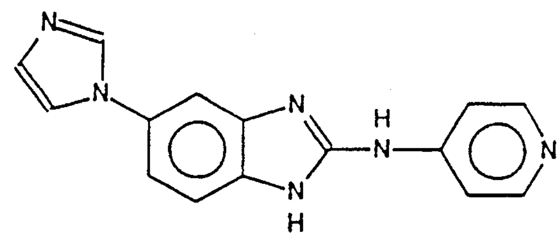
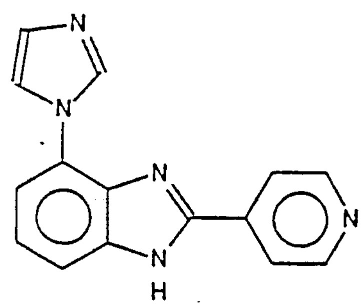
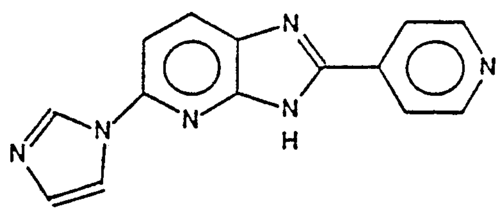
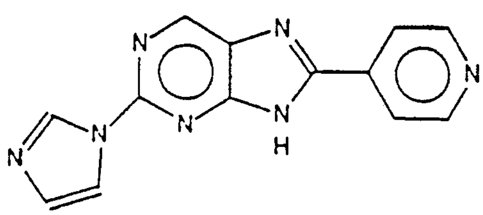
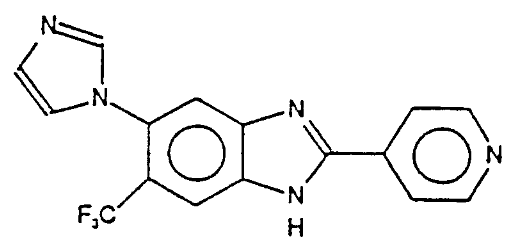
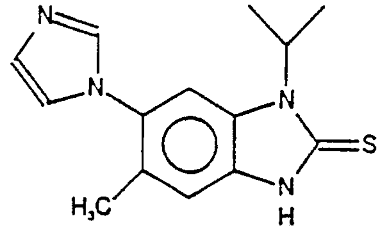
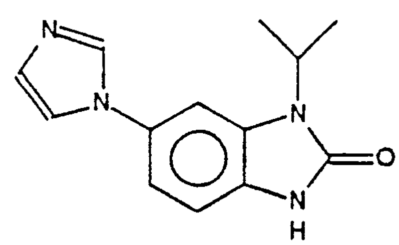

.HCL

18. Procédé de préparation d'un nouveau dérivé de benzimidazole, caractérisé en ce qu'on prépare le composé de formule chimique développée suivante :

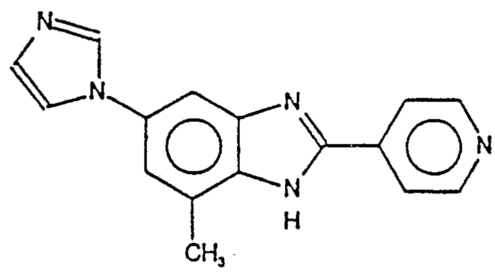

19. Procédé selon l'une quelconque des revendications 1 à 18, caractérisé en ce qu'on prépare ledit composé par réaction de l'urée, de la thiourée, du sulfure de carbone, d'un aldéhyde benzénique ou pyridinique suivi d'une oxydation, d'un chlorure d'acide benzénique ou pyridinique, d'un acide benzoïque ou pyridinique, d'un nitrile benzénique ou pyridinique, d'un dithiocarbamate benzénique ou pyridinique, d'un isothiocyanate benzénique ou pyridinique ou d'un imidodithiocarbonate benzénique ou pyridinique sur des composés diaminés de formule (II) :

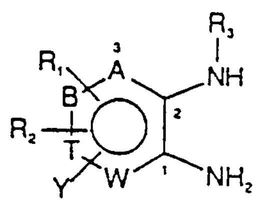

FORMULE (II)

dans laquelle

$R_1$ et $R_2$ représentent simultanément ou non l'atome d'hydrogène l'atome d'halogène ou un groupement $CF_3$, $NO_2$, $NHR_4$, $NHCOR_4$, $OR_4$,$SR_4$ ($R_4$ représentant un atome d'hydrogène ou un radical alkyle inférieur) ou un alkyle inférieur.

$R_3$ représente l'atome d'hydrogène, un alkyle inférieur ou un groupement benzyle.

Y représente un dérivé de l'imidazole,du benzimidazole,du triazole ou de l'imidazothiazole pouvant être ou non substitué par des groupements :

Halogène, $COR_5$, $OR_5$, $SR_5$, $COOR_5$ ($R_5$ étant un atome d'hydrogène ou un radical alkyle inférieur), un radical alkyle inférieur substitué ou non par des groupements halogènes, $OR_5$, $SR_5$, $COOR_5$, $NHR_5$, $NHCOR_5$, $COR_5$, $R_5$ étant défini comme ci-dessus.

20. Procédé de préparation d'une composition pharmaceutique, caractérisé en ce qu'on incorpore une quantité thérapeutiquement efficace d'au moins un composé de formule (I) tel que précédemment défini, à l'une des revendications 1 à 18, ainsi qu'éventuellement ses sels d'addition d'acides pharmacologiquement acceptables, dans un excipient, véhicule ou support pharmaceutiquement acceptable.

21. Procédé de préparation selon la revendication 20 caractérisé en ce qu'on prépare une composition pharmaceutique à activité cardiovasculaire, cardiotonique, vasodilatatrice, anti-hypertensive et anti-agrégante plaquettaire ou anti-sécrétoire ou anti-ulcère.